# EUROPEAN PATENT APPLICATION

(11) **EP 3 778 624 A1**
(43) Date of publication of application: **17.02.2021**
(21) Application number: 19786200.6
(22) Date of filing: 09.04.2019
(51) Int. Cl.: C07K 14/14, C12N 9/16, C12N 15/46, A61K 35/765, A61K 38/46, A61P 3/08

(54) **METHOD FOR PRODUCING GLUCOSE-6-PHOSPHATASE 2 PROTEIN**

(30) Priority: 09.04.2018 ES 201830351
(71) Applicant: Universidade de Santiago de Compostela, 15782 Santiago de Compostela, La Coruña (ES)
(72) Inventor: MARTÍNEZ COSTAS, José Manuel, 15782 Santiago de Compostela, A Coruña (ES); VARELA CALVIÑO, Rubén, 15782 Santiago de Compostela, A Coruña (ES); BARREIRO PIÑEIRO, Natalia, 15782 Santiago de Compostela, A Coruña (ES); BENAVENTE MARTÍNEZ, Francisco Javier, 15782 Santiago de Compostela, A Coruña (ES)
(74) Representative: ABG Intellectual Property Law, S.L.
(86) International application number: PCT/ES2019/070241
(87) International publication number: WO 2019/197697

(57) **Abstract**

The invention relates to a fusion protein comprising glucose-6-phosphatase 2 protein and a polypeptide derived from avian or mammalian Orthoreovirus muNS protein, as well as a method for producing said protein, and to the use of said protein to treat type 1 diabetes.

## Description

### FIELD OF THE INVENTION

The present invention relates to the field of recombinant protein production. Specifically, the invention relates to the production of glucose-6-phosphatase 2 protein in the form of a fusion protein with the avian Orthoreovirus muNS protein intercoil domain.

### BACKGROUND OF THE INVENTION

Obtaining both eukaryotic and prokaryotic membrane proteins is an extremely difficult process. The amount of these proteins in cells is usually rather small, such that the only way to obtain large amounts of said proteins for various applications, such as for function and structure determination or for use in immunological studies, involves cloning same into various vectors and introducing said vectors in organisms, such as bacteria or yeasts, which allow large amounts to be expressed. However, most of these proteins are toxic once their expression is induced in different organisms, greatly limiting the possibility of obtaining significant amounts of said proteins.

Glucose-6-phosphatase-2 protein (Islet-specific glucose-6-phosphatase catalytic subunit-related protein, IGRP) is an important antigen in the development of type 1 diabetes (T1D) or insulin-dependent diabetes. In this disease, the immune system of diabetic patients specifically destroys insulin-producing cells in the pancreatic islets of Langerhans. For said destruction to take place, the lymphocytes of these patients are activated by different proteins or antigens expressed by insulin-producing cells such as insulin itself, the 65 kDa isoform of glutamic acid decarboxylase (GAD65), insulinoma-associated antigen 2 (IA-2), or more recently IGRP, an antigen that was identified following its discovery as the target recognized by a highly diabetogenic murine CD8 T lymphocyte, clone 8.3 (Lieberman SM et al,. Proc Natl Acad Sci USA. 2003. 100:8384-8388). More importantly, the number of lymphocytes specific for this antigen in the peripheral blood of NOD (Non-Obese Diabetic) mice, the human T1D murine model, is capable of predicting the development of the clinical symptoms of the disease (Trudeau JD et al., J Clin Invest. 2003, 111:217-223) and the presence of CD8 T lymphocytes specific for this antigen in the pancreas of diabetic NOD mice is the most prevalent population in the first stages of islet transplant rejection (Wong CP et al., J Immunol. 2006, 176:1637-1644). Besides the NOD mouse, this antigen is also a target of immune response in diabetic human patients (Jarchum I et al., Clin Immunol. 2008, 127:359-365), making it necessary to produce and purify said antigen so as to be able to conduct studies, among other things, relating to the autoimmune response in diabetic patients or even to the possible use thereof in the future as a vaccine that may prevent disease development.

Studies concerning which segments (peptides) are recognized during the autoimmune response, necessary for designing possible vaccines based on said segments (peptides) in the future, have been greatly limited by the inability to produce significant amounts of IGRP. Furthermore, the effectiveness of a therapy in preventing the development of the clinical symptoms of the disease cannot be verified in animal models, such as the NOD mouse, due to the absence of a purified protein.

To this day there are hardly any publications which disclose the expression and purification of this protein. Martin *et al.* disclose the cloning of the open reading frame corresponding to murine IGRP and the expression of said protein as a fusion protein with beta-galactosidase, indicating that the protein is found in inclusion bodies which, according to the authors, is purified by means of preparative electrophoresis and used to obtain antibodies by means of rabbit immunization (Martin CC et al., Genes J. Biol. Chem. 2001, 276: 25197-207). Petrolonis *et al.* describe the expression and purification of IGRP with the baculovirus system (Petrolonis AJ et al., J. Biol. Chem. 2004, 279: 13976-83). None of these papers describes the production of IGRP in significant amounts.

There is therefore a need for new methods for the expression and purification of IGRP protein.

### SUMMARY OF THE INVENTION

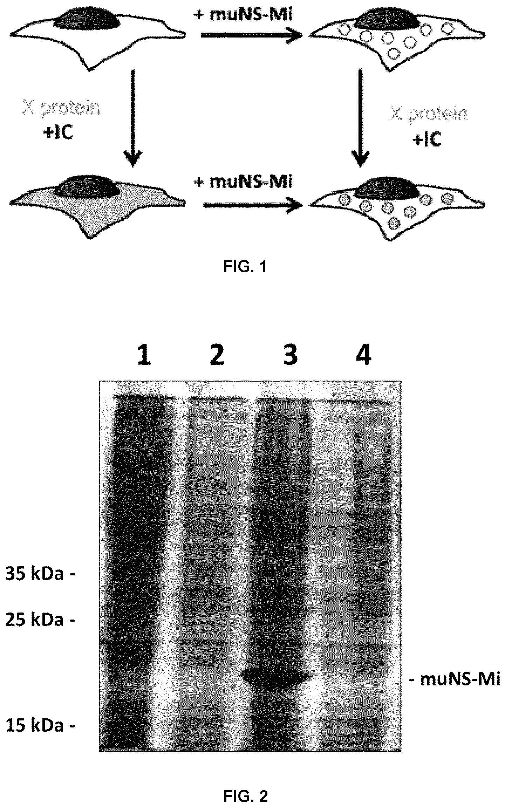

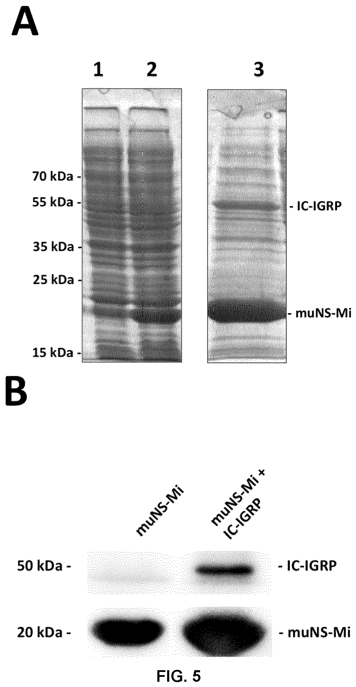

The authors of the present invention have developed a method for expressing and purifying glucose-6-phosphatase 2 protein (IGRP) as a fusion protein with the avian Orthoreovirus muNS protein intercoil (IC) domain. This system is based on the co-expression of this IGRP-IC fusion protein together with avian Orthoreovirus muNS protein-derived muNS-Mi protein, giving rise to the expression of the fusion protein in microspheres (Figure 1). Surprisingly, the inventors have verified that, unlike what happens when using other expression systems, such as the baculovirus system, it is possible to obtain microspheres in which said fusion protein is found in detectable amounts when the IGRP-IC fusion protein and the muNS-Mi protein are co-expressed in bacteria (Figure 5). This expression system has the additional advantage that the fusion protein can be purified, being separated from the microspheres by means of incubation with a buffer suitable for destructuring the microspheres. Furthermore, if the IC domain is to be eliminated, the fusion protein can be designed including a target sequence for proteases between the IGRP protein and IC. Once the fusion protein is separated from the microspheres, the IC domain can thus be separated by means of proteolysis with the specific protease. Microsphere reconstitution by means of incubation with the suitable buffer will capture the IC domain, therefore being a simple and scalable IGRP protein purification method.

Therefore, in a first aspect, the invention relates to a fusion protein comprising:
(i) glucose-6-phosphatase 2 protein (IGRP) or a functionally equivalent variant thereof, and
(ii) a polypeptide selected from the group consisting of:
   - sequence 477-542 (SEQ ID NO: 1) of avian Orthoreovirus muNS protein,
   - the sequence of mammalian Orthoreovirus muNS protein corresponding to sequence 477-542 (SEQ ID NO: 1) of avian Orthoreovirus muNS protein, and
   - a functionally equivalent variant of any of the foregoing having the capacity to be incorporated into microspheres.

In another aspect, the invention relates to a microsphere comprising a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell,
wherein the microsphere further comprises the fusion protein of the invention.

In another aspect, the invention relates to a polynucleotide encoding the fusion protein of the invention, an expression cassette comprising said polynucleotide, or a vector comprising said polynucleotide or said expression cassette.

In another aspect, the invention relates to a cell comprising the fusion protein of the invention, the polynucleotide of the invention, the expression cassette of the invention, or the vector of the invention.

In another aspect, the invention relates to a method for producing the fusion protein of the invention which comprises:
(a) expressing in a bacterial cell a first polynucleotide encoding said fusion protein and a second polynucleotide encoding a polypeptide selected from the group consisting of:
   - a polypeptide comprising amino acids 448-635 (SEQ ID NO: 2) of avian Orthoreovirus muNS protein,
   - the region of mammalian Orthoreovirus muNS protein corresponding to the region of avian Orthoreovirus muNS protein comprising amino acids 448-635 (SEQ ID NO: 2) of said protein,
   - the complete avian Orthoreovirus muNS protein,
   - the complete mammalian Orthoreovirus muNS protein, and
   - a functionally equivalent variant of any of the foregoing which maintains the capacity to form microspheres when expressed in a cell
(b) subjecting said bacterial cell to conditions suitable for the formation of microspheres, and
(c) concentrating the microspheres.

In another aspect, the invention relates to a fusion protein obtainable according to the method for producing the fusion protein of the invention.

In another aspect, the invention relates to a method for producing IGRP protein or a functionally equivalent variant thereof, which comprises the following steps:
(a) producing a fusion protein according to the method for producing the fusion protein of the invention, wherein the fusion protein comprises a protease recognition sequence between components (i) and (ii) thereof,
(b) subjecting the microspheres to conditions leading to their disintegration, with the fusion protein and the microspheres being caused to separate,
(c) contacting the product resulting from step (b) with a protease specific for the recognition sequence connecting components (i) and (ii) of the fusion protein under conditions suitable for the proteolysis of said fusion protein, with the subsequent separation of components (i) and (ii) of the fusion protein,
(d) subjecting the product of step (c) to conditions suitable for the formation of microspheres, and
(e) separating the microspheres from the IGRP protein or the functionally equivalent variant thereof.

In another aspect, the invention relates to IGRP protein or a functionally equivalent variant thereof obtainable according to the method according to the preceding aspect.

In another aspect, the invention relates to a pharmaceutical composition comprising the microsphere of the invention and a pharmaceutically acceptable excipient.

In another aspect, the invention relates to the microsphere of the invention for use in medicine.

In another aspect, the invention relates to the microsphere of the invention for use in the treatment and/or prevention of type 1 diabetes.

In another aspect, the invention relates to the use of the microsphere of the invention for the preparation of a medicinal product for the treatment and/or prevention of type 1 diabetes.

In another aspect, the invention relates to a method for inducing type 1 diabetes in an animal model which comprises administering to an animal an effective amount of the microsphere of the invention.

### BRIEF DESCRIPTION OF THE FIGURES

**Figure 1****.** Diagram of the basic operation of the "IC-Tagging" methodology.
**Figure 2****.** IGRP expression with IC-Tagging using baculovirus/Sf9 cells - PAGE analysis of the protein composition of the total extracts of Sf9 insect cells that are uninfected (1), or infected with recombinant baculoviruses directing the expression of IGRP protein (2) and muNS-Mi protein (3), or co-infected with both (4). The gel was stained with Coomassie blue.

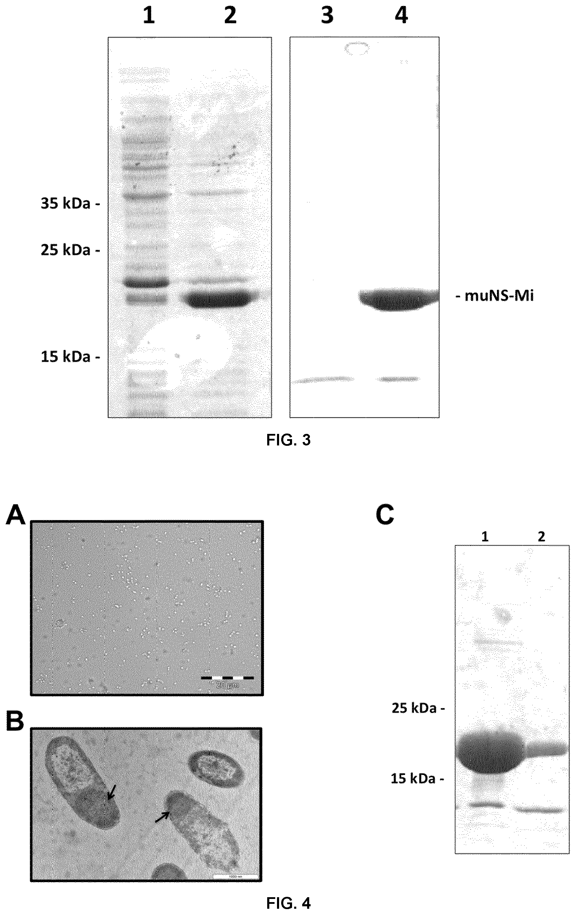

**Figure 3****.** Bacterial expression of muNS-Mi with pET Duet 1 plasmid - PAGE analysis of the protein composition of the total extracts of bacteria transformed with pET Duet-1 plasmid to express muNS-Mi protein not induced (1) or induced for three hours with IPTG imM (2). The right part of the figure shows the supernatant (3) and the pellet (4) resulting from the purification of the muNS-Mi microspheres present in the extracts, such as that shown in 2, by means of centrifugation. The gels were stained with Coomassie blue.
**Figure 4****.** muNS-Mi forms ordered microspheres in bacteria. A. Optical microscope images obtained with purified muNS-Mi samples, such as that shown in Figure 3, lane 4. B. Electron microscope images of bacteria containing muNS-Mi microspheres (indicated with an arrow) therein, after the expression thereof has been induced with IPTG. C. PAGE analysis of the supernatant (1) and the pellet (2) obtained after incubating the microspheres shown in A in a magnesium-free buffer.
**Figure 5****.** Concentration of microspheres containing IC-IGRP. A. PAGE analysis of the protein composition of the protein extracts of bacteria transformed with pET Duet-1 plasmid simultaneously expressing muNS-Mi and IC-IGRP proteins. The gel shows extracts of bacteria that are not induced (1), induced for three hours with 1 mM of IPTG (2), or induced and concentrated by means of centrifugation (3). The gels were stained with Coomassie blue. The positions of the molecular weight markers are indicated on the left and the positions of the indicated proteins on the right. B. The sample of lane 3 of A was analyzed by means of Western-blot using a specific anti-muNS antibody (right lane), comparing it with a similar sample containing only muNS-Mi (left lane).

### DETAILED DESCRIPTION

### Fusion protein and microsphere of the invention

In a first aspect, the invention relates to a fusion protein comprising:
(i) glucose-6-phosphatase 2 protein (IGRP) or a functionally equivalent variant thereof, and
(ii) a polypeptide selected from the group consisting of:
   - sequence 477-542 (SEQ ID NO: 1) of avian Orthoreovirus muNS protein,
   - the sequence of mammalian Orthoreovirus muNS protein corresponding to sequence 477-542 (SEQ ID NO: 1) of avian Orthoreovirus muNS protein, and
   - a functionally equivalent variant of any of the foregoing having the capacity to be incorporated into microspheres.

As it is used herein, the term "fusion protein" refers to polypeptides comprising two or more regions originating from different or heterologous proteins.

The fusion protein of the invention comprises two components.

### (i) Glucose-6-phosphatase 2 protein (IGRP) or a functionally equivalent variant thereof

As it is used herein, the term "glucose-6-phosphatase 2," or "IGRP," or "islet-specific glucose-6-phosphatase catalytic subunit-related protein" refers to a protein capable of hydrolyzing glucose-6-phosphate to yield glucose and phosphate. It is a transmembrane protein expressed in the pancreas and, to a lesser extent, the testicles. In humans, it is encoded by the G6PC2 gene. The IGRP protein can be of any origin, for example human, bovine, murine, equine, canine, etc. In a particular embodiment, the IGRP protein is the human protein identified by UniprotKB database accession number Q9NQR9 (version of entry 123, 25 October 2017, version of sequence 1, 1 October 2000). Three isoforms of the protein, identified by the following UniprotKB accession numbers, are found in humans:
- Isoform 1, considered the canonical sequence, with a length of 355 amino acids: Q9NQR9-1.
- Isoform 2, with a length of 102 amino acids: Q9NQR9-2.
- Isoform 3, with a length of 154 amino acids: Q9NQR9-3.

In another particular embodiment, the IGRP protein is the mouse protein identified by UniprotKB database accession number Q9Z186 (Version of entry 123, 25 October 2017; version of sequence 1, 1 May 1999). Two isoforms of the protein, identified by the following UniprotKB accession numbers, are found in mice:
- Isoform 1, considered the canonical sequence, with a length of 355 amino acids: Q9Z186-1.
- Isoform 2, with a length of 154 amino acids: Q9Z186-2.

These isoforms, as well as any other isoform of the IGRP protein of any origin, are considered as being included within the term "IGRP" according to the present invention.

In another particular embodiment, the IGRP protein is the rat protein encoded by the gene identified by NCBI Genbank database accession number Gene ID 681817 (version of 26 September 2017).

As it is used herein, the term "functionally equivalent variant" refers to any peptide or protein resulting from the deletion, insertion, addition, or substitution of one or more amino acid residues with respect to the sequence from which it is derived and which conserves the function of said sequence.

In a particular embodiment, the functionally equivalent variant has a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% in the entire length thereof with the IGRP protein sequence. The degree of identity between the variants and IGRP is determined using algorithms and computing methods that are widely known by those skilled in the art. The identity between two amino acid sequences is preferably determined using the BLASTP algorithm [BLAST Manual, Altschul, S., et al., NCBI NLM NIH Bethesda, Md. 20894, Altschul, S. et al., J. Mol Biol, 215: 403 - 410 (1990)]. In a more particular embodiment, the sequence identity between the functionally equivalent variant and the IGRP protein sequence is calculated along the entire length of the polypeptide.

In a particular embodiment, the functionally equivalent IGRP variants comprise additions of at least 1, at least 2, at least 3, at least 4, at least 5, at least 6, at least 7, at least 8, at least 9, at least 10, at least 15, at least 20 amino acids. In a particular embodiment, the functionally equivalent variants comprise additions of less than 20, less than 15, less than 10, less than 9, less than 8, less than 7, less than 6, less than 5, less than 4, less than 3, less than 2, or 1 amino acid. Similarly, variants comprising deletions of at least 1, at least 2, at least 3, at least 4, at least 5, at least 6, at least 7, at least 8, at least 9, at least 10, at least 15, at least 20 amino acids are also contemplated. In a particular embodiment, the functionally equivalent variants comprise deletions of less than 20, less than 15, less than 10, less than 9, less than 8, less than 7, less than 6, less than 5, less than 4, less than 3, less than 2, or 1 amino acid. Functionally equivalent variants having a substitution of at least 1, at least 2, at least 3, at least 4, at least 5, at least 6, at least 7, at least 8, at least 9, at least 10, at least 15, at least 20 amino acids of the IGRP protein sequence are also contemplated. In a particular embodiment, the functionally equivalent variants comprise substitutions of less than 20, less than 15, less than 10, less than 9, less than 8, less than 7, less than 6, less than 5, less than 4, less than 3, less than 2, or 1 amino acid. In a particular embodiment, the functionally equivalent variants comprise conservative substitutions of one or more amino acids. As it is used herein, the term "conservative substitution" refers to replacing one amino acid with another having similar structural and/or chemical properties. For example, the following six groups each contains amino acids that are conservative substitutions of one another: 1) alanine (A), serine (S), threonine (T); 2) aspartic acid (D), glutamic acid (E); 3) asparagine (N), glutamine (Q); 4) arginine (R), lysine (K); 5) isoleucine (I), leucine (L), methionine (M), valine (V); and 6) phenylalanine (F), tyrosine (Y), tryptophan (W)

The functionally equivalent variants of IGRP may have amino acids additions, deletions, and substitutions in any position. In a particular embodiment, the functionally equivalent variants of IGRP comprise amino acids additions at the N-terminal end, at the C-terminal end, or at both ends of the IGRP sequence. In another particular embodiment, the functionally equivalent variants of IGRP comprise amino acid deletions at the N-terminal end, at the C-terminal end, or at both ends of the IGRP sequence.

In a particular embodiment, the functionally equivalent variant of the IGRP protein conserves at least 50%, at least 60%, at least 70%, at least 80%, at least 90%, at least 95%, at least 100% of the function of the IGRP protein.

The IGRP protein is capable of inducing diabetes when administered to a mouse in the form of a DNA vaccine (Fuchs et al., Clinical and Experimental Immunology, 2014, 176: 199-206). In a particular embodiment, the functionally equivalent variants of the IGRP protein conserve the capacity to induce diabetes of the IGRP protein from which they are derived. The capacity of a protein to induce diabetes in an animal model can be determined by means of methods known to one skilled in the art, for example, by means of determining urine or blood glucose levels. For example, Fuchs *et al.* (*supra*) considered that the murine model has diabetes when two consecutive measurements of urine glucose levels above 5.5 mmol/L or blood glucose levels above 13.9 mmol/L are obtained. Furthermore, the IGRP protein exhibits glucose 6-phosphatase activity, i.e., it is capable of catalyzing the dephosphorylation of glucose-6-phosphate to glucose. In a particular embodiment, the functionally equivalent variants of the IGRP protein conserve the phosphatase activity of the IGRP protein from which they are derived. The glucose-6-phosphatase activity can be determined by means of methods known to one skilled in the art, for example, methods based on the detection of inorganic phosphate in aqueous solutions, such as the malachite green method (Petrolonis *et al., supra*)*,* or methods based on the detection of glucose-6-phosphate, such as the methods based on the glucose oxidase/peroxidase system (Mao, H., et al., Anal. Chem. 2002, 74, 379-385), for example.

### (ii) Polypeptide

The second component of the fusion protein of the invention is a polypeptide selected from the group consisting of:
- sequence 477-542 (SEQ ID NO: 1) of avian Orthoreovirus muNS protein,
- the sequence of mammalian Orthoreovirus muNS protein corresponding to sequence 477-542 (SEQ ID NO: 1) of avian Orthoreovirus muNS protein, and
- a functionally equivalent variant of any of the foregoing having the capacity to be incorporated into microspheres.

In a particular embodiment, the polypeptide which is part of the fusion protein of the invention consists of sequence 477-542 (SEQ ID NO: 1) of avian Orthoreovirus muNS protein.

As it is used herein, the term "avian Orthoreovirus" or "avian Reovirus" refers to one of the 12 genera belonging to the family of *Reoviridae* virus, and specifically to the group within the genus that infects birds. They have double-stranded RNA genomes and therefore belong to virus group III.

As it is used herein, the term "avian Orthoreovirus muNS protein" refers to one of the non-structural proteins encoded by avian Reovirus or avian Orthoreovirus M3 gene and it is the only avian Reovirus protein capable of forming microspheres when expressed in the absence of other factors (Touris-Otero et al. Virology, 319; 94-1069). It is a 635-amino acid protein defined by NCBI database accession number Ay608700 (version Ay608700.1, 7 August 2004) or by NCBI database accession number AAS78998 (version AAS78998.1, 10 April 2006). In a particular embodiment, avian Orthoreovirus muNS protein has the sequence SEQ ID NO: 3.

Sequence 477-542 (SEQ ID NO: 1) of avian Orthoreovirus muNS protein is an avian Orthoreovirus muNS protein fragment referred to as "intercoil" or "IC". Said fragment has the capacity to be incorporated into microspheres formed by avian Orthoreovirus muNS protein or the minimum fragment of said protein.

As it is used herein, the term "microsphere" or "inclusion" refers to nuclear or cytoplasmic, usually protein, aggregates. Specifically, the protein forming the microspheres in the genus *Orthoreovirus* is the muNS or µNS protein, which is one of the non-structural proteins encoded by the M3 gene and the only avian Reovirus protein capable of forming microspheres when expressed in the absence of other viral factors (Touris-Otero *et al., supra*)*.*

In another particular embodiment, the polypeptide which is part of the fusion protein of the invention consists of the sequence of mammalian Orthoreovirus muNS protein corresponding to sequence 477-542 (SEQ ID NO: 1) of avian Orthoreovirus muNS protein.

As it is used herein, the term "mammalian Orthoreovirus" refers to one of the 12 genera belonging to the family of *Reoviridae* virus, and specifically to the group of the genus that infects mammals. They have double-stranded RNA genomes and therefore belong to virus group III.

As it is used herein, the term "mammalian Orthoreovirus muNS or µNS protein" refers to one of the non-structural proteins encoded by mammalian Reovirus or mammalian Orthoreovirus and is the only mammalian Reovirus protein capable of forming microspheres when expressed in the absence of other viral factors (Becker, M. M. et al. 2003. J. Virol. 77:5948-5963). It is a 721-amino acid protein defined by NCBI database accession number ABP48918 (version ABP48918.1, 11 April 2008) (SEQ ID NO: 4).

To determine the corresponding sequence of mammalian Orthoreovirus muNS protein with respect to said avian Orthoreovirus muNS protein fragments, an alignment between the sequence of the avian muNS protein and the sequence of the mammalian Orthoreovirus muNS protein can be carried out. Said sequence alignment can be carried out by means of conventional methods known to one skilled in the art. Optimal sequence alignments can be carried out by means of, for example, the Smith and Waterman local homology algorithm (Adv. Appl. Math., 1981, 2:482), the Needleman and Wunsch homology alignment algorithm, (J. Mol. Biol., 1970, 48:443), the Pearson and Lipman search for similarity method, (Proc. Natl Acad. Sci. USA, 1988, 85:2444), computerized implementation of these algorithms (GAP, BESTFIT, FASTA, and TFASTA in the Wisconsin Genetics Software Package, Genetics Computer Group, 575 Science Dr., Madison, WI), or manual alignment and visual inspection (Current Protocols in Molecular Biology (Ausubel et al, eds. 1995 supplement).

In a particular embodiment, the sequence of mammalian Orthoreovirus muNS protein corresponding to sequence 477-542 (SEQ ID NO: 1) of avian Orthoreovirus muNS protein is sequence 561-622 (SEQ ID NO: 5) of mammalian Orthoreovirus muNS protein.

In a particular embodiment, the polypeptide which is part of the fusion protein of the invention consists of a functionally equivalent variant of (i) sequence 477-542 (SEQ ID NO: 1) of avian Orthoreovirus muNS protein or (ii) the sequence of mammalian Orthoreovirus muNS protein corresponding to sequence 477-542 (SEQ ID NO: 1) of avian Orthoreovirus muNS protein, having the capacity to be incorporated into microspheres.

"Functionally equivalent variant" is understood to be all those peptides derived from sequence 477-542 (SEQ ID NO: 1) of avian Orthoreovirus muNS protein or from the sequence of mammalian Orthoreovirus muNS protein corresponding to sequence 477-542 (SEQ ID NO: 1) of avian Orthoreovirus muNS protein by means of modification, insertion, substitution, and/or deletion of one or more amino acids, provided that the function of the sequences of the muNS protein from which they are derived is substantially maintained.

Sequence 477-542 (SEQ ID NO: 1) of avian Orthoreovirus muNS protein and the sequence of mammalian Orthoreovirus muNS protein corresponding to sequence 477-542 (SEQ ID NO: 1) of avian Orthoreovirus muNS protein has the capacity to be incorporated into microspheres formed by the complete muNS protein or the minimum region of said protein with the capacity to form microspheres (muNS-Mi) in a cell. The functionally equivalent variants of sequence 477-542 (SEQ ID NO: 1) of avian Orthoreovirus muNS protein and the sequence of mammalian Orthoreovirus muNS protein corresponding to sequence 477-542 (SEQ ID NO: 1) of avian Orthoreovirus muNS protein substantially conserve the capacity of said sequences to be incorporated into the microspheres formed by the complete protein or muNS-Mi in a cell. Methods suitable for determining the capacity of being incorporated into microspheres include, but are not limited to, the method described in Example 3 of patent application WO 2011/098652 based on the formation of the microspheres (inclusions) and the expression of the protein of interest in the form of a fusion protein associated with fragments directing same into the microspheres. Indirect immunofluorescence using polyclonal antibodies specific against the HA epitope or the epitope of interest would be carried out thereafter, where the incorporation of said fragments into the microspheres can be verified.

The functionally equivalent variants of sequence 477-542 (SEQ ID NO: 1) of avian Orthoreovirus muNS protein or of the sequence of mammalian Orthoreovirus muNS protein corresponding to sequence 477-542 (SEQ ID NO: 1) of avian Orthoreovirus muNS protein have the capacity to be incorporated into microspheres. In a particular embodiment, the functionally equivalent variants of sequence 477-542 (SEQ ID NO: 1) of avian Orthoreovirus muNS protein or of the sequence of mammalian Orthoreovirus muNS protein corresponding to sequence 477-542 (SEQ ID NO: 1) of avian Orthoreovirus muNS protein conserve at least 50%, at least 60%, at least 70%, at least 80%, at least 90%, at least 95%, at least 100% of the capacity to be incorporated into microspheres of the sequences from which they are derived.

The functionally equivalent variants of sequence 477-542 (SEQ ID NO: 1) of avian Orthoreovirus muNS protein or of the sequence of mammalian Orthoreovirus muNS protein corresponding to sequence 477-542 (SEQ ID NO: 1) of avian Orthoreovirus muNS protein include those showing at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

The two components of the fusion protein can be connected in any order, i.e., component (ii) can be fused to the amino terminal end of component (i) or component (i) can be fused to the amino terminal end of component (ii). In a particular embodiment, component (ii) is fused to the amino terminal end of component (i).

In a particular embodiment, the two components of the fusion protein are connected through a protease recognition sequence, thereby allowing the separation of the two components. The protease recognition sequences suitable for being incorporated in the fusion protein of the invention include enterokinase (cleavage site DDDDK - SEQ ID NO:6), factor Xa (cleavage site IEDGR - SEQ ID NO:7), thrombin (cleavage site LVPRGS - SEQ ID NO:8), TEV protease (cleavage site ENLYFQG - SEQ ID NO:9), PreScission protease (cleavage site LEVLFQGP - SEQ ID NO:10), inteins, and the like. In a particular embodiment, the protease recognition sequence is an enterokinase recognition sequence or a factor Xa recognition sequence. In a more particular embodiment, the protease recognition sequence is the sequence of SEQ ID NO: 6 or the sequence of SEQ ID NO: 7.

In a particular embodiment, the fusion protein does not exhibit any eukaryotic post-translational modifications. As it is used herein, the term "post-translational modifications" refers to the modification of proteins, once synthesized, through the addition of a chemical group by means of a covalent bond. As it is used herein, the term "eukaryotic post-translational modifications" refers to the post-translational modifications occurring in eukaryotic organisms and not in prokaryotic organisms. Non-limiting illustrative examples of eukaryotic post-translational modifications include: myristoylation, palmitoylation, farnesylation, geranylgeranylation, modification with glycosylphosphatidylinositol (GPI), acylation, acetylation, alkylation, methylation, and glycosylation.

In another aspect, the invention relates to a microsphere comprising a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell,
wherein the microsphere comprises the fusion protein of the invention.

The terms "microsphere," "avian Orthoreovirus muNS protein," and "mammalian Orthoreovirus muNS protein" have been described above.

The microsphere of the invention comprises or is formed by a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell.

The term "polypeptide comprising amino acids 448-635 (SEQ ID NO: 2) of avian Orthoreovirus muNS protein" or "muNS-Mi" refers to the indicated region of avian Orthoreovirus muNS protein. Said region is the minimum region of avian Orthoreovirus muNS protein having the capacity to form microspheres when expressed in a cell.

The region of mammalian Orthoreovirus muNS protein corresponding to the region of avian Orthoreovirus muNS protein comprising amino acids 448-635 (SEQ ID NO: 2) of said protein can be determined as explained above for the region of mammalian Orthoreovirus muNS protein corresponding to sequence 477-542 (SEQ ID NO: 1) of avian Orthoreovirus muNS protein.

In a particular embodiment, the sequence of mammalian Orthoreovirus muNS protein corresponding to sequence 448-635 (SEQ ID NO: 2) of avian Orthoreovirus muNS protein is sequence 518-721 (SEQ ID NO: 11) of mammalian Orthoreovirus muNS protein.

Sequence 448-635 (SEQ ID NO: 2) of avian Orthoreovirus muNS protein, the sequence of mammalian Orthoreovirus muNS protein corresponding to sequence 448-635 (SEQ ID NO: 2) of avian Orthoreovirus muNS protein, and the complete avian Orthoreovirus muNS protein and complete mammalian Orthoreovirus muNS protein have the capacity to form microspheres when expressed in a cell. The functionally equivalent variants of these sequences substantially conserve the capacity of said sequences to form microspheres. Methods suitable for determining the capacity to form microspheres include, but are not limited to, the method described in Example 1 of patent application WO 2011/098652, in which microsphere (inclusion) formation is detected by means of indirect immunofluorescence microscopy using anti-muNS polyclonal antibodies.

The functionally equivalent variants of sequence 448-635 (SEQ ID NO: 2) of avian Orthoreovirus muNS protein, the sequence of mammalian Orthoreovirus muNS protein corresponding to sequence 448-635 (SEQ ID NO: 2) of avian Orthoreovirus muNS protein, and the complete avian Orthoreovirus muNS protein and complete mammalian Orthoreovirus muNS protein have the capacity to form microspheres.

In a particular embodiment, the functionally equivalent variants of sequence 448-635 (SEQ ID NO: 2) of avian Orthoreovirus muNS protein, the sequence of mammalian Orthoreovirus muNS protein corresponding to sequence 448-635 (SEQ ID NO: 2) of avian Orthoreovirus muNS protein, and the complete avian Orthoreovirus muNS protein and complete mammalian Orthoreovirus muNS protein conserve at least 50%, at least 60%, at least 70%, at least 80%, at least 90%, at least 95%, at least 100% of the capacity to form microspheres of the sequences from which they are derived.

The functionally equivalent variants of sequence 448-635 (SEQ ID NO: 2) of avian Orthoreovirus muNS protein, the sequence of mammalian Orthoreovirus muNS protein corresponding to sequence 448-635 (SEQ ID NO: 2) of avian Orthoreovirus muNS protein, and the complete avian Orthoreovirus muNS protein and complete mammalian Orthoreovirus muNS protein include those showing at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity with respect to the sequences from which they are derived.

The microsphere of the invention can be obtained by means of the methods described herein.

### Polynucleotide, expression cassette, vector, and cell of the invention

In another aspect, the invention relates to a polynucleotide encoding the fusion protein of the invention.

As it is used herein, the term "polynucleotide" refers to a polymer formed by a variable number of monomers, wherein the monomers are nucleotides, including both ribonucleotides and deoxyribonucleotides. The polynucleotides include monomers modified by means of methylation, as well as non-modified forms. The terms "polynucleotide" and "nucleic acid" are used interchangeably herein and include mRNA, cDNA, and recombinant polynucleotides.

In another aspect, the invention relates to an expression cassette comprising the polynucleotide of the invention.

As it is used herein, the term "expression cassette" refers to a polynucleotide comprising a gene and a promoter suitable for controlling that gene. The expression cassette may optionally include other sequences, for example, transcription termination signals. The choice of a promoter and other regulatory element(s) generally varies depending on the host cell used. Suitable promoters in the context of the present invention include constitutive promoters which promote the expression of sequences associated therewith in a constant manner and inducible promoters which require an external stimulus to promote the transcription of sequences associated therewith.

Promoters useful for the embodiment of the present invention include:
- Constitutive promoters such as, for example, alcohol dehydrogenase (ADH1) promoter, elongation factor 1-alpha (TEF) promoter, and triose phosphate isomerase (TPI) encoding gene promoter, glyceraldehyde 3-phosphate dehydrogenase (GPD) promoter, and 3-phosphoglycerate kinase (GPK) promoter, MRP7 promoter.
- Inducible promoters such as, for example, metallothionein (CUP1) promoter the expression of which is regulated by means of the addition of copper to the culture medium, FUS1 gene- or FUS2 gene-encoding gene promoter the expression of which is activated in the presence of pheromones (factor α), TET promoter the expression of which is regulated in the presence of tetracyclines, GAL1-10, GALL, GALS promoters which are activated in the presence of galactose, VP16-ER inducible promoter by estrogens, phosphatase (PH05) promoter the expression of which is activated in the presence of phosphate, and HSP150 heat shock protein promoter the expression of which is activated at high temperature.

In a particular embodiment, when the cell in which the polynucleotide of the invention will be expressed is a bacterium, said promoter is the beta-lactamase and lactose promoter system, T7 RNA polymerase promoter, lambda promoter, trp promoter, or tac promoter. In a more particular embodiment of the invention, said promoter is an inducible promoter. In another more particular embodiment, said promoter is an isopropyl-β-D-1-thiogalactopyranoside (IPTG) inducible promoter. In an even more particular embodiment, the IPTG inducible promoter is T7 RNA polymerase promoter. In another aspect, the invention relates to a vector comprising the polynucleotide or the expression cassette of the invention.

As it is used herein, the term "vector" refers to a nucleic acid sequence comprising the sequences necessary for the generation of the fusion protein of the invention after the transcription and translation of said sequences in a cell. Said sequence is operatively bonded to additional segments providing for the autonomous replication thereof in a host cell of interest. Preferably, the vector is an expression vector which is defined as a vector which, in addition to the autonomous replication regions in a host cell, contains regions that are operatively ligated to the polynucleotide of the invention and are capable of enhancing the expression of the products of the polynucleotide according to the invention. The vectors of the invention can be obtained by means of techniques widely known in the art. In a particular embodiment of the invention, when the organism is a prokaryote, such as a bacterium, suitable vectors according to the invention are, for example, vectors pUC18, pUC19, pUC118, pUC119, Bluescript and derivatives thereof, mp18, mp19, pBR322, pMB9, ColEI, pCRI, RP4, pNH8A, pNH16a, pNH18a. In a particular embodiment of the invention, when said bacterium is *E. coli,* said vector is pET plasmid, in particular pET Duet-1 plasmid. Said plasmid comprises two different multiple cloning sites (MSC). The genes cloned into said plasmid are transcribed under the control of bacteriophage T7 promoter, when T7 RNA polymerase is activated in the host cell. Expression is induced with IPTG (isopropyl-β-D-thiogalactopyranoside), which removes the repressor of the operator so that transcription is carried out and expression of the protein of interest is promoted.

In a particular embodiment, the vector of the invention further comprises a second polynucleotide encoding a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell.

The terms "avian Orthoreovirus muNS protein" and "mammalian Orthoreovirus muNS protein" have been described above. The polypeptide comprising amino acids 448-635 (SEQ ID NO: 2) of avian Orthoreovirus muNS protein, the region corresponding to said polypeptide of mammalian Orthoreovirus muNS protein, as well as the functionally equivalent variants, have been defined above.

In a particular embodiment, the polynucleotide of the invention is operatively bonded to a promoter and the second polynucleotide is operatively bonded to a second promoter. The particular embodiments of the first promoter are likewise applicable to the second promoter. In a particular embodiment, the first and second promoters are inducible promoters. In another more particular embodiment, the first and second promoters are isopropyl-β-D-1-thiogalactopyranoside (IPTG) inducible promoters. In an even more particular embodiment, the IPTG inducible promoter is T7 RNA polymerase promoter.

In another aspect, the invention relates to a cell comprising the fusion protein, the polynucleotide, the expression cassette, or the vector of the invention.

The cell of the invention can be any prokaryotic cell or any eukaryotic cell. In a particular embodiment, the cell is a prokaryotic cell. In a more particular embodiment, the cell is a bacterial cell. In an even more particular embodiment, the bacterial cell is *Escherichia coli.* Examples of *E. coli* strains that can be used to propagate the polynucleotides or vectors of the invention or to produce the fusion protein of the invention include strains JM109, DH5α™, XL-1 Blue, or BL21 DE3.

### Method for producing the fusion protein of the invention

In another aspect, the invention relates to a method for producing the fusion protein of the invention which comprises:
(a) expressing in a bacterial cell a first polynucleotide encoding said fusion protein and a second polynucleotide encoding a polypeptide selected from the group consisting of:
   - a polypeptide comprising amino acids 448-635 (SEQ ID NO: 2) of avian Orthoreovirus muNS protein,
   - the region of mammalian Orthoreovirus muNS protein corresponding to the region of avian Orthoreovirus muNS protein comprising amino acids 448-635 (SEQ ID NO: 2) of said protein,
   - the complete avian Orthoreovirus muNS protein,
   - the complete mammalian Orthoreovirus muNS protein, and
   - a functionally equivalent variant of any of the foregoing which maintains the capacity to form microspheres when expressed in a cell
(b) subjecting said bacterial cell to conditions suitable for the formation of microspheres, and
(c) concentrating the microspheres.

All the terms have been described above. The particular embodiments of said terms likewise apply to the method for producing the fusion protein of the invention.

The first step of the method for producing the fusion protein of the invention comprises expressing in a bacterial cell a first polynucleotide encoding said fusion protein and a second polynucleotide encoding a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell.

As known to one skilled in the art, in order to express the first and second polynucleotides in a bacterial cell, both polynucleotides must be introduced in the bacterial cell, for example, by means of transforming said cell with vectors comprising each of the polynucleotides or with a vector comprising both polynucleotides. If the first and second polynucleotides are part of independent vectors, both vectors may be introduced in the cell simultaneously or sequentially.

If one or both polynucleotides are operatively bonded to an inducible promoter, to express said polynucleotides, the bacterial cell will have to be contacted with the inducer. In the particular case of one or both promoters being IPTG inducible promoters, for the expression of the polynucleotides, IPTG is added to the bacterial culture for the time necessary until both polynucleotides are expressed, for example at least 30 minutes, at least 1 hour, at least 2 hours, at least 3 hours, at least 4 hours, at least 5 hours, at least 6 hours, at least 12 hours, at least 18 hours, at least 24 hours, at least 48 hours, at least 72 hours, or more. Preferably, induction with IPTG is performed for an approximate time period of 3 hours. In a particular embodiment, the cells are incubated during induction at a temperature between 18 and 37°C, preferably between 25 and 37°C, more preferably 37°C. In a particular embodiment, when the induction time is more than 24 hours, the cells are incubated during induction at a temperature between 18 and 25°C. Once the induction has ended, the bacteria are collected by centrifugation.

If the first and second polynucleotides are operatively bonded to different promoters, both polynucleotides can be expressed simultaneously if they can be expressed under the same conditions, or sequentially. If the first and second polynucleotides are operatively bonded to different inducible promoters, both polynucleotides may be expressed simultaneously by adding both inducers at the same time to the culture medium of the cells, or sequentially by adding one of the inducers first and then the other.

The second step of the method for producing the fusion protein of the invention consists of subjecting the bacterial cells to conditions suitable for the formation of microspheres.

The conditions suitable for the formation of microspheres can be determined by one skilled in the art for each cell type. Methods suitable for detecting the formation of microspheres include, but are not limited to, the method described in Example 1 of patent application WO 2011/098652, in which microsphere (inclusion) formation is detected by means of indirect immunofluorescence microscopy using anti-muNS polyclonal antibodies. In a particular embodiment, the conditions suitable for the formation of microspheres comprise incubating the cells expressing the first and second polynucleotides for a time period of at least 30 minutes, at least 1 hour, at least 2 hours, at least 3 hours, at least 4 hours, at least 5 hours, at least 6 hours, at least 12 hours, at least 18 hours, at least 24 hours, and at a temperature between 25 and 37°C, preferably 37°C.

The first and second steps of the method for producing the fusion protein of the invention can be carried out simultaneously or sequentially, such that the first and second polynucleotides are first expressed, and the cells are then subjected to conditions suitable for the formation of microspheres. In a particular embodiment, the first and second steps are carried out simultaneously, given that the conditions to which the bacterial cells are subjected for the expression of the first and second polynucleotides are suitable for the formation of microspheres. In a more particular embodiment, said conditions comprise incubating the cells in the presence of the inducer, preferably IPTG, for a time period of at least 30 minutes, at least 1 hour, at least 2 hours, at least 3 hours, at least 4 hours, at least 5 hours, at least 6 hours, at least 12 hours, at least 18 hours, at least 24 hours, and at a temperature between 25 and 37°C, preferably 37°C.

The third step of the method for producing the fusion protein of the invention consists of concentrating the microspheres. To concentrate the microspheres, the bacterial cells in which the microspheres have been formed must be lysed by means of any method, such as incubation with a lysis buffer or sonication, among others. The microspheres can then be pelleted by means of centrifugation, preferably at a speed of about 2700 g. In a particular embodiment, once the cells are lysed, the microspheres are washed with a buffer comprising a divalent cation. As it is used herein, the term "divalent cation" refers to a positively charged ion of any metal from the periodic table which has a valence of 2. Divalent cations suitable for use in the present invention include, without limitation, divalent Mg, Cd, Ca, Co, Cu, Fe, Mn, Ni, Sr, and Zn cations. In a preferred embodiment, the divalent cation is Mg²⁺. Divalent cation concentrations suitable for inducing muNS protein aggregate formation are, for example, at least 0.5 mM, at least 0.8 mM, at least 1 mM, at least 5 mM, at least 10 mM, at least 15 mM, at least 20 mM, or higher. In a particular embodiment, the microspheres are washed with a buffer comprising Mg²⁺, for example MgCl₂, preferably at a concentration of 5 mM. In an even more particular embodiment, the microspheres are concentrated following the purification protocol indicated in the examples herein.

In a particular embodiment, the method for producing the fusion protein of the invention further comprises purifying the fusion protein by subjecting the microspheres to conditions leading to their disintegration.

The conditions leading to the disintegration of the microspheres include, among others:
- incubation of the microspheres with denaturing agents, for example, urea or guanidinium hydrochloride,
- incubation of the microspheres with ionic detergents, for example, SDS at a high concentration,
- incubation with NaCl at concentrations above 500 mM,
- incubation with a buffer not comprising divalent cations, preferably not comprising Mg²⁺.

In a particular embodiment, the conditions leading to the disintegration of the microspheres are incubation with a buffer not comprising divalent cations, preferably not comprising Mg²⁺.

In a particular embodiment, once the microspheres have disintegrated, the fusion protein can be purified by means of any known method.

In another aspect, the invention relates to a fusion protein obtainable according to the method for producing the fusion protein of the invention.

In another aspect, the invention relates to a fusion protein of the invention, wherein said protein lacks eukaryotic post-translational modifications.
The term "eukaryotic post-translational modifications" has been described above.

### Method for producing IGRP protein

In another aspect, the invention relates to a method for producing IGRP protein or a functionally equivalent variant thereof, which comprises the following steps:
(a) producing a fusion protein according to the method for producing a fusion protein of the invention, wherein the fusion protein comprises a protease recognition sequence between components (i) and (ii) thereof,
(b) subjecting the microspheres to conditions leading to their disintegration, with the fusion protein and the microspheres being caused to separate,
(c) contacting the product resulting from step (b) with a protease specific for the recognition sequence connecting components (i) and (ii) of the fusion protein under conditions suitable for the proteolysis of said fusion protein, with the subsequent separation of components (i) and (ii) of the fusion protein,
(d) subjecting the product of step (c) to conditions suitable for the formation of microspheres, and
(e) separating the microspheres from the IGRP protein or the functionally equivalent variant thereof.

In a particular embodiment, the protease recognition sequence joining components (i) and (ii) of the fusion protein is an enterokinase recognition sequence (cleavage site DDDDK - SEQ ID NO: 6), factor Xa (cleavage site IEDGR - SEQ ID NO: 7), thrombin (cleavage site LVPRGS - SEQ ID NO: 8), TEV protease (cleavage site ENLYFQG - SEQ ID NO: 9), PreScission protease (cleavage site LEVLFQGP - SEQ ID NO: 10), inteins, or the like. In a more particular embodiment, the protease recognition sequence is an enterokinase recognition sequence or a factor Xa recognition sequence. In an even more particular embodiment, the protease recognition sequence is the sequence of SEQ ID NO: 6 or the sequence of SEQ ID NO: 7.

Steps (a) and (b) of the method for producing IGRP protein or a functionally equivalent variant thereof have been described above.

Conditions suitable for the proteolysis of the fusion protein comprise contacting the fusion protein with the protease specific for the recognition sequence connecting components (i) and (ii) of the fusion protein under conditions of pH, temperature, etc. that will depend on the specific protease to be used. Determination of these conditions for each particular protease is within the knowledge of one skilled in the art.

Conditions suitable for the formation of microspheres include contacting the product resulting from step (a) with a buffer containing a divalent cation, as described above. In a preferred embodiment, the divalent cation is Mg²⁺. Divalent cation concentrations suitable for inducing muNS protein aggregate formation are, for example, at least 0.01 mM, at least 0.1 mM, at least 1 mM, at least 2 mM, at least 3 mM, at least 4 mM, at least 5 mM, or higher. In a particular embodiment, said conditions comprise incubation with a buffer comprising Mg²⁺, preferably at a concentration of 5 mM.

According to the present method, when the microspheres form again, component (ii) of the fusion protein is integrated back into said microspheres, component (i) of the fusion protein remaining free. Therefore, this is a method suitable for the purification of IGRP or the functionally equivalent variant thereof.

In another aspect, the invention relates to IGRP protein or a functionally equivalent variant thereof obtainable according to the method for obtaining IGRP or a functionally equivalent variant thereof of the invention.

In another aspect, the invention relates to IGRP protein or a functionally equivalent variant thereof, wherein said protein or said variant lacks eukaryotic post-translational modifications.

The term "eukaryotic post-translational modifications" has been described above.

### Pharmaceutical composition

Microspheres are potent immune response inducers. The inventors have verified in an assay with bluetongue virus (BTV) epitopes that vaccination with muNS-Mi microspheres loaded with three BTV epitopes (VP2, VP7, and NS1) protects against a lethal challenge with the virus in the absence of any added adjuvant, whereas vaccination with microspheres without said epitopes, or with the epitopes alone without being integrated in microspheres, does not provide any protection whatsoever (Marin-Lopez, A. et al., VP2, VP7, and NS1 proteins of bluetongue virus targeted in avian reovirus muNS-Mi microspheres elicit a protective immune response in IFNAR(-/-) mice. Antiviral Research 110, 42-51 (2014)).

Moreover, it has been described that IGRP-derived peptides, as well as modifications thereof, are capable of preventing the development of type 1 diabetes in a murine model (Han, B. et al., Nature Medicine 2005, 11:645-652) and that the use of complete antigens, peptides, or modifications derived from these autoantigens allows inducing tolerance instead of a pro-inflammatory response under specific conditions (Clemente-Casares X et al. Cold Spring Harb Perspect Med 2012, 2(2): a007773).

Therefore, the microspheres of the invention can be used as a vaccine to induce IGRP antigen tolerance and to therefore prevent type 1 diabetes.

Therefore, in another aspect, the invention relates to a pharmaceutical composition comprising the microsphere of the invention and a pharmaceutically acceptable excipient.

As it is used herein, the expression "pharmaceutical composition" refers to a formulation which has been adapted for the administration of a predetermined dose of one or more useful therapeutic agents to a cell, a group of cells, an organ, a tissue, or an animal.

As it is used herein, the expression "therapeutically effective amount" is understood to be an amount that is capable of providing a therapeutic effect and can be determined by one skilled in the art through commonly used means. In particular, the therapeutically effective amount of the microspheres of the invention is the amount capable of indicating IGRP tolerance in the patient. The amount of microspheres of the invention which can be included in the pharmaceutical compositions according to the invention will vary depending on the subject and the particular mode of administration. Those skilled in the art will observe that dosages can also be determined with the guidance of Goodman and Goldman's The Pharmacological Basis of Therapeutics, 9th edition (1996), Appendix II, pages 1707-1711 and Goodman and Goldman's The Pharmacological Basis of Therapeutics, 10th Edition (2001), Appendix II, pages 475-493.

The pharmaceutical compositions of the invention also contain one or more pharmaceutically acceptable excipients. "Pharmaceutically acceptable excipient" is understood to be a therapeutically inactive substance which is used to incorporate the active ingredient and is acceptable for the patient from a pharmacological/toxicological viewpoint and for the pharmaceutical chemist producing same from a physical/chemical viewpoint with respect to composition, formulation, stability, patient acceptance, and bioavailability. The excipient or vehicle also includes any substance serving to improve the administration and the effectiveness of the active ingredient within the pharmaceutical composition. Examples of pharmaceutically acceptable vehicles include one or more of water, saline solution, phosphate-buffered saline solution, dextrose, glycerol, ethanol, and the like, as well as combinations thereof. In many cases, it will be preferable to include isotonic agents, for example, sugars, polyalcohols such as mannitol, sorbitol, or sodium chloride, in the composition. The pharmaceutically acceptable vehicles may further comprise smaller amounts of auxiliary substances, such as wetting or emulsifying agents, preservatives, or buffers, which increase the shelf life or the effectiveness of the microspheres or compositions that are part of the pharmaceutical compositions. Examples of suitable vehicles are well known in the literature (see, for example, Remington's Pharmaceutical Sciences, 19th ed., Mack Publishing Company, Easton, PA, 1995). In some cases, a disintegrant such as cross-linked polyvinylpyrrolidone, agar, alginic acid, or sodium alginate can be added. The number and nature of the pharmaceutically acceptable excipients depend on the desired dosage form. Pharmaceutically acceptable excipients are known to those skilled in the art (Faulí and Trillo C. (1993) "Tratado de Farmacia Galenica", Luzán 5, S.A. Ediciones, Madrid).

The pharmaceutical compositions of the invention can be administered though any suitable route, such as oral, subcutaneous, intravenous, intraperitoneal, or intramuscular route.

### Medical uses of the invention

In another aspect, the invention relates to the microsphere of the invention for use in medicine.

In another aspect, the invention relates to the microsphere of the invention for the treatment and/or prevention of type 1 diabetes. Alternatively, the invention relates to the microsphere of the invention for the preparation of a medicinal product for the treatment and/or prevention of type 1 diabetes. Alternatively, the invention relates to a method for preventing type 1 diabetes which comprises administering a therapeutically effective amount of the microsphere of the invention to a patient at risk of developing type 1 diabetes.

As it is used herein, the term "type 1 diabetes," or "diabetes mellitus type I," or "juvenile diabetes," or "insulin-dependent diabetes mellitus" refers to a metabolic disease characterized by a selective destruction of pancreatic beta cells causing an absolute insulin deficiency. It differs from type 2 diabetes in that it is a type of diabetes characterized by the occurrence thereof in the early stage of life, generally before 30 years of age. Only 1 out of every 20 diabetic people has type 1 diabetes, which occurs more frequently in young people and children. Type 1 diabetes is classified into autoimmune cases, the most common form, and idiopathic cases. As it is used herein, the term "type 1 diabetes" includes both classic type 1 diabetes, generally diagnosed before 30 years of age and requiring insulin treatment from the moment of diagnosis, and latent autoimmune diabetes of the adult, diagnosed after 30 years of age and not usually requiring insulin treatment within 3-6 months after diagnosis.

In a particular embodiment, the type 1 diabetes is autoimmune type 1 diabetes. As it is used herein, the term "autoimmune type 1 diabetes" refers to an autoimmune disease involving a selective destruction of pancreatic β cells mediated by T lymphocytes activated in subjects with predisposing HLA haplotypes. After a preclinical period of variable duration during which the patient remains asymptomatic, when the mass of insulin-producing cells reaches a critical value, the patient exhibits the classic symptomatology: polyuria, polydipsia, polyphagia, weight loss, and a progressive ketosis that may end in ketoacidosis, if no exogenous insulin treatment is established.

In a more particular embodiment, as it is used herein, the type 1 diabetes is "latent autoimmune diabetes of the adult" or "LADA," which refers to a type of autoimmune diabetes diagnosed in adulthood, normally after 30 years of age, in subjects who are positive for at least one autoantibody from among those normally present in patients with classic type 1 diabetes, for example, islet cell antibodies (ICA), glutamic acid decarboxylase antibodies (GADA), islet antigen-2 antibodies (IA-A2), or insulin autoantibodies (IAA), and who do not require insulin treatment within the first 3 or 6 months after diagnosis. Autoimmune diabetes of the adult differs from type 1 diabetes in terms of the slowly progressive β cell failure and the resulting gradual insulin dependence. In patients with LADA, β cell function is usually affected within six years following diagnosis.

As it is used herein, the term "treatment" refers to the capacity of the microsphere of the invention to alleviate or eliminate the disease, type 1 diabetes, or to reduce or eliminate one or more symptoms associated with type 1 diabetes.

As it is used herein, the term "prevention" refers to the capacity of the microsphere of the invention to prevent, minimize, or hinder the development of type 1 diabetes in a patient.

As it is used herein, the term "patient" or "subject" refers to any animal, preferably a mammal, and includes, among others, domestic and farm animals, primates, and human beings, for example, human beings, non-human primates, cows, horses, pigs, sheep, goats, dogs, cats, or rodents such as rats and mice. In a preferred embodiment, the subject is a human being of any age or race. In a particular embodiment, the subject is at risk of developing type 1 diabetes. A subject at risk of developing type 1 diabetes is that individual who has an immediate family member (a brother/sister) diagnosed with type 1 diabetes and who furthermore has multiple (≥ 2) serum islet cell antibodies (ICA), glutamic acid decarboxylase 65 antibodies (GADA65), islet antigen-2 antibodies (IA-2), or ZnT8 transporter (ZnT8).

The term "therapeutically effective amount" has been described above. In a particular embodiment, the therapeutically effective amount is that which successfully conserves serum peptide C levels, and/or conserves/reduces glycosylated hemoglobin (HbA1c) levels, and/or reduces the mean insulin dosage, and/or induces the generation of regulatory T lymphocytes T. "Therapeutically effective amount" refers to the amount of antigen administered with the microspheres, which can be determined by means of conventional protein quantification methods.

In a particular embodiment, the route of administration of the microspheres of the invention is oral, subcutaneous, intravenous, intraperitoneal, or intramuscular.

In a particular embodiment, the microspheres are administered in the absence of an adjuvant. Without wishing to be bound to a particular theory, it is considered that the administration of antigens in the absence of adjuvant favors the development of a tolerance response in the organism, instead of an inflammatory response. As it is used herein, the term "adjuvant" refers to an immunological agent modifying the effect of an immunogen, while having few, if any, direct effects when it is administered alone. It is often included in vaccines to increase the immune response of the receptor to the supplied antigen, while at the same time maintaining the injected exogenous material at a minimum. Adjuvants are added to vaccines to stimulate the response of the immune system to the target antigen, but they themselves do not confer immunity. Non-limiting examples of useful adjuvants include mineral salts, polynucleotides, polyarginines, ISCOM, saponins, monophosphoryl lipid A, imiquimod, CCR5 inhibitors, toxins, polyphosphazenes, cytokines, immunoregulatory proteins, immunostimulatory fusion proteins, costimulatory molecules, and combinations thereof. The mineral salts include, but are not limited to, AlK(SO₄)₂, AlNa(SO₄)₂, AlNH₄(SO₄), silica, alum, Al(OH)₃, Ca₃(PO₄)₂, kaolin, or carbon. Useful immunostimulatory polynucleotides include, but are not limited to, CpG oligonucleotides with or without immunostimulatory complexes (ISCOM), CpG oligonucleotides with or without polyarginine, poly IC or poly AU acids. The toxins include cholera toxin. The saponins include, but are not limited to, QS21, QS17, or QS7. An example of a useful immunostimulatory fusion protein is the IL-2-immunoglobulin Fc fragment fusion protein. Useful immunoregulatory molecules include, but are not limited to, CD40L and CD1a ligand. Cytokines useful as adjuvants include, but are not limited to, IL-1, IL-2, IL-4, GMCSF, IL-12, IL-15, IGF-1, IFN-α, IFN-β, and interferon gamma. Furthermore, examples of adjuvants are muramyl dipeptides, such as N-acetyl-muramyl-L-threonyl-D-isoglutamine (thr-DMP), N-acetyl-normuramyl-L-alanyl-D-isoglutamine (CGP 11687, also referred to as nor-MDP), N-acetylmuramyl-L-alanyl-D-isoglutaminyl-L-alanine-2-(1'2'-dipalmitoyl-sn-glycero-3-hydr oxyphosphoryloxy)-ethylamine (CGP 19835A, also referred to as MTP-PE), RIBI (MPL+TDM+CWS) in a 2% emulsion of squalene/TWEEN® 80, lipopolysaccharides and several derivatives thereof, including lipid A, complete Freund's adjuvant (CFA), incomplete Freund's adjuvant, Merck Adjuvant 65, polynucleotides (for example, poly IC and poly AU acids), wax D of *Mycobacterium tuberculosis,* substances found in *Corynebacterium parvum, Bordetella pertussis,* and members of the genus *Brucella,* Titermax, Quil A, ALUN, lipid A derivatives, cholera toxin derivatives, HSP derivatives, LPS derivatives, MPL derivatives, GMDP or synthetic peptide matrices, Montanide ISA-51 and QS-21, CpG oligonucleotide, poly I:C, and GMCSF.

### Method for inducing diabetes in an animal model

It has been described above that the administration of a DNA vaccine including the IGRP protein coding sequence to NOD mice accelerates the development of type 1 diabetes (Fuchs, V.F. et al., Clin Exp Immunol 2014, 176(2):199-206).

Therefore, in another aspect, the invention relates to a method for inducing type 1 diabetes in an animal model which comprises administering an effective amount of the microsphere of the invention to said animal.

The term "type 1 diabetes" has been defined above. In a particular embodiment, the type 1 diabetes is an autoimmune diabetes. In a particular embodiment, the type 1 diabetes is latent autoimmune diabetes of the adult.

As it is used herein, the term "animal model" refers to a test animal that suffers from a disease or may develop a disease similar to said disease in humans, and can be used as the model for studying said disease. An animal model must be simple and reproducible. Furthermore, the disease of the animal model must have the same etiology as in humans, or progress in a pattern similar to that of humans. Accordingly, mammalian vertebrates having a body structure similar to that of humans, for example with respect to internal organs, immune system, and body temperature, and suffering from diseases such as high blood pressure, cancer, immunodeficiency, etc., are suitable as animal models. The animals of the models are particularly mammals, such as horses, sheep, pigs, goats, camels, antelopes, dogs, rabbits, mice, rats, guinea pigs, and hamsters, and more particularly rodents such as mice, rats, guinea pigs, and hamsters. A mouse is often used in the study of human diseases as a result of its many advantages, including its small size, high fertility, easy handling in terms of feeding, high resistance to diseases, hereditary uniformity, and strain variety. Another advantage for accepting the use thereof as an animal model is that mice can be transformed to exhibit diseases or symptoms identical or similar to those of human beings.

In a particular embodiment, the animal model is a mouse or murine model.

In a more particular embodiment, the animal is genetically modified to favor the development of type 1 diabetes. Example of animals, particularly mice, genetically modified to favor the development of diabetes are transgenic mice expressing B7-1 and a viral glycoprotein in their pancreatic beta cells together with T cells expressing the viral glycoprotein-specific transgenic T-cell receptor (Harlan, D.M. et al., Proc Natl Acad Sci USA 1994, 91(8): 3137-41) or transgenic mice expressing the viral glycoprotein (GCV) of lymphocytic choriomeningitis (GPM) in pancreatic islet beta cells (Ohashi, P.S. et al., Cell 1991, 65(2): 305-17).

When the animal model is a murine model, the microsphere preferably comprises murine IGRP protein.

As it is used herein, the term "effective amount" refers to the amount of microspheres required to induce an immune response against the IGRP protein, and ultimately the development of diabetes. In a particular embodiment, when the animal is a mouse, the effective amount of microspheres is the amount comprising between 1 and 5 µg of IGRP protein/animal.

In a particular embodiment, the administration is performed subcutaneously or intramuscularly.

In a particular embodiment, the microspheres are administered together with an adjuvant. The term "adjuvant" has been defined above. In a more particular embodiment, the adjuvant is a complete Freund's adjuvant. As it is used herein, the term "complete Freund's adjuvant" or "CFA" refers to a mineral oil solution comprising dry inactivated mycobacteria, generally (*M. tuberculosis*)*.*

The invention is described below by means of the following examples that must be considered merely illustrative and in no way limit the scope of the present invention.

### Examples

### Materials and methods

### Cells and bacteria

Sf-9 insect cells were grown by means of suspension culture at 28°C in SF-900 II medium (Thermo Fisher) supplemented with 5% FBS, 2% glutamine, and 1% antibiotics (Thermo Fisher). The bacteria used for obtaining the bacmid were DH10Bac (Invitrogen) which were heat-shock transformed and then seeded in LB agar plates with gentamicin (7 µg/ml) (Sigma-Aldrich, Madrid, Spain), tetracycline (10 µg/ml) (Sigma-Aldrich, Madrid, Spain), and kanamycin (50 µg/ml). The bacteria used for plasmid propagation were XL1-Blue (Stratagene), which were likewise heat-shock transformed and seeded in LB agar plates with ampicillin (100 µg/ml) (Sigma-Aldrich, Madrid, Spain). The bacteria used for protein expression through T7 promoter were BL21 DE3 C+ (Sigma-Aldrich, Madrid, Spain).

### Cloning and obtainment of recombinant baculoviruses

To obtain the baculovirus which expresses IC-IGRP fusion protein, the IGRP sequence from the complementary DNA marketed by Ambion (PCR-Ready Human Pancreas cDNA) was amplified by means of PCR using the following primers: sense 5'-GCGAAGCTTGGCATGGATTTCCTTCACAGGAATGG - 3' (SEQ ID NO: 12) and antisense 5'- GCGAAGCTTCTACTGACTCTTCTTTCCGCTTTGTTTC -3' (SEQ ID NO: 13) (target *Hind*III underlined, stop codon double underlined). The resulting PCR was subjected to digestion with the Hindlll enzyme and then ligated into pFastBac-1 plasmid, obtaining pFastBac IGRP plasmid. To introduce the tag IC sequence before the IGRP, the IC sequence from a pGEMT-M3 plasmid (Brandariz-Nuñez, A. et al., PLoS ONE 2010, 5, e13785) was amplified by means of PCR using the following primers: sense 5'-GCATAAGAATGCGGCCGCTATCATGGCGGAAGATCACTTGTTGGCTTATC -3' (SEQ ID NO: 14) and antisense 5'-GCGTCTAGACGCTTCCACACGGGGTTCCCACTCAG -3' (SEQ ID NO: 15) (target *Not*I and *Xba*I underlined, ATG initiator double underlined). The PCR product was digested with *Not*I and *Xba*l enzymes and cloned into pFastBac IGRP plasmid to create pFastBac IC-IGRP plasmid. The sequences were confirmed by means of DNA sequencing (STABVIDA, Portugal).

The obtainment of the bacmid from DH10Bac bacteria and subsequently baculovirus by means of transfecting Sf9 cells with recombinant bacmids was performed as recommended by the provider of the bac-to-bac system (Invitrogen).

To verify the expression of proteins with the recombinant baculoviruses, Sf-9 cells seeded in a single layer at 600,000 cells/ml were infected with said viruses. The cells were collected 3 days post infection and centrifuged at 16000X g for 2 minutes.

They were washed 2 times with 1X PBS and loaded in a polyacrylamide gel that was subsequently stained with Coomassie blue.

### Adaptation of the IC-Tagging system to bacteria

pET Duet-1 dual expression plasmid having two MCS under the control of T7 polymerase was used for protein expression in bacteria. First, the muNS-Mi sequence was cloned into MCS1 of pET Duet-1 plasmid. To that end, the sequence was amplified by means of PCR using pGEMT-M3 plasmid as template (Touris-Otero, F. et al., J Mol Biol 2004, 341: 361-374) and the following primers: sense 5'-CATGCCATGGCACCAGCCGTACTGCTGTC-3' (SEQ ID NO: 16) and antisense 5'-TTGCGGCCGCAATCACAGATCATCCACC -3' (SEQ ID NO: 17) (raget *N*coI and *Not*I underlined and stop codon double underlined). The amplification product was digested with *Nco*I and *Not*I enzymes and ligated into MCS1 of pET Duet-1 plasmid to generate pET Duet-1 1.muNS-Mi. Then, the IC-IGRP sequence was introduced into MCS2 of pET Duet-1 1.muNS-Mi plasmid. To that end, the IC-IGRP sequence was amplified by means of PCR using the following primers: sense 5'-GGAGATCTCGCGGAAGATCACTTGTTGGC- 3' (SEQ ID NO: 18) and antisense 5'-GGGATATCCTACTGACTCTTCTTTCCGC -3' (SEQ ID NO: 19) (target *Bg*III and *EcorR*V underlined and stop codon double underlined). The PCR product was digested with the corresponding enzymes and ligated into MCS2 of pET Duet-1 1.muNS-Mi plasmid to generate pET Duet-1 1.muNS-Mi 2.IC-IGRP.

### Expression and purification of proteins in bacteria

For protein expression in bacteria, BL21 DE3 C+ bacteria were transformed with the corresponding plasmid. Once colonies were obtained, one colony was grown in LB with the corresponding antibiotic and incubated for 12 hours at 37°C under stirring. That pre-culture was then diluted 25 times in a total volume of 100 ml of LB and incubated for 2 hours and 30 minutes at 37°C under stirring. Expression was then induced with 1 mM of IPTG and incubation was performed for 3 hours at 37°C under stirring. The bacteria were collected by centrifugation at 3200X g, 30min and 4°C. The pellet was washed 2 times with 1X PBS and resuspended in a bacterial lysis buffer with lysozyme and protease inhibitor, leaving it at -20°C for at least 12 hours. The bacteria were then thawed and lysed by means of sonication and centrifuged at 2700 g for 5 minutes at 4°C, discarding the supernatant. The pellet was washed 1 time with TRB+ with 0.5% Triton X-100, 3 times with TRB+, and finally resuspended in 1 ml of TRB+. For SDS-PAGE analysis, the sample was first broken up by incubation with 10% SDS for 15 minutes at room temperature.

### Western-blot

For Western-blot analysis, the protein extracts were subjected to SDS-PAGE and the proteins were then transferred to PVDF membranes (Immobilion-P Millipore) for 1 hour at 100 mA. The proteins were detected with specific antibodies using Immobilion Western Chemiluminiscent HRP Substrate (Millipore).

### Solutions and buffers

PBS: 137 mM NaCl; 2,7 mM KCI; 8 mM Na₂PO₄ and 1.5 mM KH₂PO₄.
PBST: 137 mM NaCl; 2.7 mM KCI; 8 mM Na₂PO₄; 1.5 mM KH₂PO₄, and 0.05% Tween-20.
PBST-Milk: 137 mM NaCl; 2.7 mM KCI; 8 mM Na₂PO₄; 1.5 mM KH₂PO₄; 0.05% Tween-20, and 5% skimmed milk.
SDS-PAGE electrophoresis buffer 1X (Tris-glycine-SDS): 25 mM Tris-HCI (pH 8.3); 192 mM glycine and 0.1% SDS.
DNA sample buffer (6X): 0.25% bromophenol blue; 0.25% xylene cyanol, and 30% glycerol.
Laemmli sample buffer: 60 mM Tris-HCI (pH 6.8); 2% SDS; 5% β-mercaptoethanol; 10% glycerol, and 0.024% bromophenol blue.
Transfer buffer: 25 mM Tris-HCI (pH 8.3); 192 mM glycine, and 20% methanol. Tris-EDTA (TE) buffer: 10 mM Tris-HCI (pH 8.0) and 1 mM EDTA.
Tris-acetate-EDTA (TAE) buffer: 0.004 M Tris-acetate and 0.001 M EDTA.
TRB +: 10 mM Hepes pH 7.9; 10 mM KCI; 5 mM MgCI2.
Bacterial lysis buffer: 0.25% Tween 20, 1 mM DTT, 200 mM NaCl, 20 mM Tris pH 7.5, 2 mM MgCI2.

### Results

At first, the attempt was made to express the IGRP protein with conventional systems without using the IC-Tagging system: expression in bacteria and baculovirus/insect cells. The presence of the protein (data not shown) could not be detected in any of the cases.

Next, it was decided to try to express the protein using IC-Tagging in the baculovirus/insect cell expression system characterized by the inventors. To that end, Sf9 cells were co-infected with two recombinant baculoviruses: one expressing muNS-Mi protein and another having the IGRP protein sequence fused to the C-terminal end of the IC tag, under the control of the polyhedrin promoter. This way, said baculovirus should express the IC-IGRP fusion protein. Three days post-infection, the total extracts of infected cells were analyzed in polyacrylamide gels stained with Coomassie blue. It can be clearly seen in the gel that when the Sf9 cells were infected only with baculovirus directing the expression of muNS-Mi, a prominent band corresponding to the size of the anticipated protein appears (Figure 2, lane 3). In contrast, no expression whatsoever of IGRP protein was observed, not when the attempt was made to express it alone (Figure 2, lane 2) or in combination with muNS-Mi (Figure 2, lane 4). Furthermore, in the latter case, not only was no expression whatsoever of IGRP observed, but the inclusion of recombinant baculovirus for IGRP also caused the expression of muNS-Mi (Figure 2, lane 4) to be cancelled. Several attempts were made to optimize expression by varying the relative multiplicities of the two baculoviruses used, but such attempts were unsuccessful.

It was then decided to try to adapt the IC-tagging method to bacterial expression systems to see if the expression of this protein and other proteins could be improved. First, given that this system requires the simultaneous expression of two different proteins, it was decided to use pET Duet-1 plasmid, designed for these purposes, having two different multiple cloning sites (MCS), in which the sequences of two different proteins were introduced. Each of them is under the control of a T7 polymerase-independent promoter and the generated mRNA will have a ribosome binding site. First, the muNS-Mi sequence was introduced into one of the MCS, and construct correction was verified by means of sequencing. To verify the correct expression of the protein, a fresh culture of BL21 DE3 C+ bacteria transformed with the construct was induced with IPTG and extracts of non-induced and induced cells were analyzed by means of polyacrylamide gels. Figure 3 allows verifying the increased intensity of a kD band, corresponding to muNS-Mi, in the extracts of bacteria induced with IPTG (lane 2), in comparison with bacteria that are not induced (lane 1).

Next, it was decided to verify if muNS-Mi was capable of forming microspheres inside bacteria, like it does in eukaryotic cells. First, an adapted purification protocol was designed, said protocol consisting of: i) washing the bacteria with PBS, ii) lysing the bacteria in a typical bacterial lysis buffer (0.25% Tween 20, 1 mM DTT, 1 mg/ml lysozyme, 200 mM NaCl, and 20 mM Tris HCL pH 7.5) with the help of sonication, and iii) washing the pellets several times with a magnesium-containing buffer (10 mM Hepes pH 7.9, 10 mM KCI, 5 mM MgCl₂, and 0.5% Triton X-100) with the aid of centrifugation, finally leaving them in detergent-free washing buffer. It can be seen in lanes 3 and 4 of Figure 3 that there is hardly any protein left in the supernatants of the washes (3), whereas the final pellet shows a high degree of purification of muNS-Mi protein (4). The material shown in lane 4 in Figure 3 was also analyzed by means of microscopy. A drop of the material was placed on a slide with a coverslip arranged thereon, and it was viewed with a 100X objective under an Olympus BX51 microscope, checking for the presence of particles pushing the microscope detection limit (Figure 4A). Although there were individualized particles, most of them seemed to associate in groups of two. By analyzing this material by means of DLS (dynamic light scattering), a small peak around half a micron and a larger peak corresponding to one micron were confirmed, which coincides with individual particles or aggregates of two particles (data not shown). To achieve better characterization of these particles, it was decided to analyze the presence of microspheres inside the bacteria transformed and induced with IPTG by means of electron microscopy. Figure 4B shows representative photographs in which the presence of spherical inclusions inside the induced bacteria which are not present in the non-induced bacteria (not shown) can be seen. The observed structures have an approximate diameter of 400-500 nm, coinciding with what is observed under optical microscope and in DLS.

To rule out that the observed and purified spheres simply represent typical bacterial inclusion bodies, it was decided to exploit one of the characteristics of muNS-Mi microspheres that is not shared at all by inclusion bodies: the instability thereof in the absence of magnesium. To that end, microspheres purified from bacteria were incubated in a magnesium-free buffer for 4 hours. The samples were then centrifuged and the supernatant was separated from the pellet, analyzing both in polyacrylamide gels. It could thereby be verified that most of the protein readily solubilized in the absence of magnesium remaining in the supernatant (Figure 4C, lane 1), whereas only a residual amount remained in the pellet (Figure 4C, lane 2), which could be completely eliminated by adding more buffer (not shown).

After verifying the correct construction of the recombinant plasmid, the suitable expression of muNS-Mi, and the production of microspheres inside the bacteria, the IGRP sequence was introduced into the second MCS. Once the correct construction of the recombinant plasmid was confirmed by means of sequencing, the joint expression of muNS-Mi and IGRP was analyzed. To that end, as described above, fresh cultures of BL21 DE3 C+ bacteria transformed with the double recombinant plasmid (muNS-Mi/IC-IGRP) the expression of which was induced with IPTG were prepared. Significant expression of IGRP did not seem to be observed both after 3 hours of induction (Figure 5, comparing lane 1 with lane 2) and after 18 hours of induction at room temperature (not shown), although the presence of muNS-Mi was indeed observed in both cases, which indicated that, unlike what occurred in the attempts of expression with the baculovirus/insect cell system, the expression of IGRP did not have negative effects on the expression of muNS-Mi in bacteria. It was then decided to concentrate the microspheres produced after the induction of the double recombinant plasmid so as to try to recover the IGRP protein which could be expressed in the bacteria but which perhaps coincided with a bacterial protein or was at a concentration too low to enable detection in polyacrylamide gels without purification and/or concentration. To that end, after inducing a fresh culture of bacteria transformed with the double recombinant, the microspheres were concentrated as indicated above for the results shown in Figure 3. After concentrating the microspheres produced inside the bacteria by means of centrifugation, a band that is visible after staining with Coomassie blue was obtained, said band corresponding with the anticipated size for IGRP-IC (Figure 5, lane 3). The identity of said band was doubly confirmed by means of Western-Blot against muNS which detects the IC tag (Figure 6b, lane X) and by means of mass spectrometry (nLC-MS/MS).

### Conclusions

As shown, by using the IC-Tagging system in bacteria the inventors are capable of producing a significant amount of IGRP, unlike what occurred when the same attempt was made in the baculovirus system. This indicates that the two methods are not equivalent but complementary, both structurally, where the microspheres produced with baculovirus are larger and more stable, and functionally. With the bacterial method, proteins such as IGRP the expression of which is initially undetectable, can therefore be produced in usable amounts, opening up the possibility of producing it on a larger scale. This system furthermore allows breaking up the microspheres by means of simple incubation with a buffer if the subsequent purification of the protein is of interest. In this sense, a target for specific proteases, such as enterokinase or Factor Xa, communicating the sequence of the protein of interest (IGRP in this case) and muNS-Mi, can even be designed such that after breaking up the microsphere, the IC tag can be separated from the protein of interest by means of specific sequence proteolysis. Next, it will be sufficient to change the buffer again in order to reconstitute the sphere which will capture the tag but not the protein, constituting an extremely simple, as well as perfectly scalable, purification system.

## Claims

1. A fusion protein comprising:
(i) glucose-6-phosphatase 2 protein (IGRP) or a functionally equivalent variant thereof, and
(ii) a polypeptide selected from the group consisting of:
- sequence 477-542 (SEQ ID NO: 1) of avian Orthoreovirus muNS protein,
- the sequence of mammalian Orthoreovirus muNS protein corresponding to sequence 477-542 (SEQ ID NO: 1) of avian Orthoreovirus muNS protein, and
- a functionally equivalent variant of any of the foregoing having the capacity to be incorporated into microspheres.

2. The fusion protein according to claim 1, wherein component (ii) is fused to the amino terminal end of component (i).

3. The fusion protein according to any of claims 1 or 2, wherein components (i) and (ii) are connected through a protease recognition sequence.

4. The fusion protein according to claim 3, wherein the protease recognition sequence is an enterokinase recognition sequence or a factor Xa recognition sequence.

5. A microsphere comprising a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell,
wherein the microsphere further comprises a fusion protein according to any of claims 1 to 4.

6. A polynucleotide encoding a fusion protein according to any of claims 1 to 4.

7. An expression cassette comprising a polynucleotide according to claim 6.

8. A vector comprising a polynucleotide according to claim 6 or an expression cassette according to claim 7.

9. The vector according to claim 8, further comprising a second polynucleotide encoding a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell.

10. The vector according to claim 9, wherein the polynucleotide according to claim 5 is operatively bonded to a first promoter and the second polynucleotide is operatively bonded to a second promoter.

11. The vector according to claim 10, wherein the first and second promoters are T7 promoter.

12. The vector according to any of claims 9 to 11, wherein said vector is a bacterial expression vector.

13. A cell comprising a fusion protein according to any of claims 1 to 4, a polynucleotide according to claim 6, an expression cassette according to claim 7, or a vector according to any of claims 8 to 12.

14. A method for producing a fusion protein according to any of claims 1 to 4, which comprises:
(a) expressing in a bacterial cell a first polynucleotide encoding said fusion protein and a second polynucleotide encoding a polypeptide selected from the group consisting of:
- a polypeptide comprising amino acids 448-635 (SEQ ID NO: 2) of avian Orthoreovirus muNS protein,
- the region of mammalian Orthoreovirus muNS protein corresponding to the region of avian Orthoreovirus muNS protein comprising amino acids 448-635 (SEQ ID NO: 2) of said protein,
- the complete avian Orthoreovirus muNS protein,
- the complete mammalian Orthoreovirus muNS protein, and
- a functionally equivalent variant of any of the foregoing which maintains the capacity to form microspheres when expressed in a cell
(b) subjecting said bacterial cell to conditions suitable for the formation of microspheres, and
(c) concentrating the microspheres.

15. The method according to claim 14, wherein the first and second polynucleotides are part of one and the same vector, and wherein the first polynucleotide is operatively bonded to a first promoter and the second polynucleotide is operatively bonded to a second promoter.

16. The method according to claim 15, wherein the first and second promoters are T7 promoter.

17. The method according to any of claims 14 to 16, which further comprises purifying the fusion protein, separating it from the microspheres.

18. A fusion protein obtainable according to the method of any of claims 14 to 17.

19. A method for producing IGRP protein or a functionally equivalent variant thereof, which comprises the following steps:
(a) producing a fusion protein according to the method of any of claims 14 to 17, wherein the fusion protein comprises a protease recognition sequence between components (i) and (ii) thereof,
(b) subjecting the microspheres to conditions leading to their disintegration, with the fusion protein and the microspheres being caused to separate,
(c) contacting the product resulting from step (b) with a protease specific for the recognition sequence connecting components (i) and (ii) of the fusion protein under conditions suitable for the proteolysis of said fusion protein, with the subsequent separation of components (i) and (ii) of the fusion protein,
(d) subjecting the product of step (c) to conditions suitable for the formation of microspheres, and
(e) separating the microspheres from the IGRP protein or the functionally equivalent variant thereof.

20. An IGRP protein or a functionally equivalent variant thereof obtainable according to the method of claim 19.

21. A pharmaceutical composition comprising the microsphere according to claim 5 and a pharmaceutically acceptable excipient.

22. The microsphere according to claim 5 for use in medicine.

23. The microsphere according to claim 5 for use in the treatment and/or prevention of type 1 diabetes.

24. The microsphere for use according to claim 23, wherein the type 1 diabetes is latent autoimmune diabetes of the adult.

25. The microsphere for use according to any of claims 22 to 24, wherein the microsphere is administered subcutaneously or intravenously.

26. The microsphere for use according to any of claims 22 to 25, wherein the microsphere is administered in the absence of an adjuvant.

27. Use of the microsphere according to claim 5 for the preparation of a medicinal product for the treatment and/or prevention of type 1 diabetes.

28. Use according to claim 27, wherein the type 1 diabetes is latent autoimmune diabetes of the adult.

29. Use according to any of claims 27 or 28, wherein the microsphere is administered subcutaneously or intravenously.

30. Use according to any of claims 27 to 29, wherein the microsphere is administered in the absence of an adjuvant.

31. A method for inducing type 1 diabetes in an animal model which comprises administering to an animal an effective amount of the microsphere according to claim 5.

32. The method according to claim 31, wherein the administration is performed subcutaneously or intramuscularly.

33. The method according to any of claims 31 or 32, wherein the microsphere is administered together with an adjuvant.


SEQUENCE LISTING

<110>  UNIVERSIDADE DE SANTIAGO DE COMPOSTELA

<120>  Method for producing glucose-6-phosphatase 2 protein

<130>  P15641PC00

<150>  ES P201830351
<151>  2018-04-09

<160>  19

<170>  PatentIn version 3.5

<210>  1
<211>  66
<212>  PRT
<213>  Artificial Sequence

<220>
<223>  Avian Orthoreovirus muNS protein fragment 477-542

<400>  1

Glu Asp His Leu Leu Ala Tyr Leu Asn Glu His Val Cys Val Asn Ala
1               5                   10                  15

Lys Asp His Glu Lys Gly Leu Leu Ala Arg Cys Asn Val Ser Gly Asp
            20                  25                  30

Ser Ile Ser Ser Ile Leu Gly Gln Arg Met Lys Asn Arg Glu Arg Phe
        35                  40                  45

Glu Thr Arg Leu Arg His Glu Ala Ser Ala Glu Trp Glu Pro Arg Val
    50                  55                  60

Glu Ala
65

<210>  2
<211>  188
<212>  PRT
<213>  Artificial Sequence

<220>
<223>  Avian Orthoreovirus muNS protein fragment 448-635

<400>  2

Pro Ala Ala Leu Leu Ser Lys Ile Ala Asp Leu Gln Arg Ala Asn Arg
1               5                   10                  15

Glu Leu Ser Leu Lys Leu Val Asp Val Gln Pro Ala Arg Glu Asp His
            20                  25                  30

Leu Leu Ala Tyr Leu Asn Glu His Val Cys Val Asn Ala Lys Asp His
        35                  40                  45

Glu Lys Gly Leu Leu Ala Arg Cys Asn Val Ser Gly Asp Ser Ile Ser
    50                  55                  60

Ser Ile Leu Gly Gln Arg Met Lys Asn Arg Glu Arg Phe Glu Thr Arg
65                  70                  75                  80

Leu Arg His Glu Ala Ser Ala Glu Trp Glu Pro Arg Val Glu Ala Leu
                85                  90                  95

Asn Gln Glu Leu Ala Lys Ala Arg Val Glu Gln Gln Asp Met Met Thr
            100                 105                 110

Gln Ser Leu Gln Tyr Leu Asn Glu Arg Asp Glu Leu Leu Gln Glu Val
        115                 120                 125

Asp Glu Leu Lys Arg Glu Leu Thr Thr Leu Arg Ser Ala Asn Val Arg
    130                 135                 140

Leu Asn Ala Asp Asn His Arg Met Ser Arg Ala Thr Arg Val Gly Asp
145                 150                 155                 160

Ala Phe Val Ser Asp Val Glu Pro Leu Pro Ser Gly Ile Pro Gly Glu
                165                 170                 175

Ser Lys Pro Ser Met Glu Glu Leu Val Asp Asp Leu
            180                 185

<210>  3
<211>  635
<212>  PRT
<213>  Avian Orthoreovirus

<400>  3

Met Ala Ser Thr Lys Trp Gly Asp Lys Pro Met Ser Val Ser Met Ser
1               5                   10                  15

His Asp Gly Ser Ser Ile Arg Ser Ala Ala Ser Gln Phe Leu Ser Gly
            20                  25                  30

Pro Leu Phe His Ser Thr Pro Ile Pro Pro Gln Arg Lys Thr Val Leu
        35                  40                  45

Leu Lys Phe Met Ile Gly Asp Glu Leu Val Thr Val Gln Gly Ala Leu
    50                  55                  60

Ala Pro Phe Asp Glu Tyr Trp Tyr Asp Asn Gln Pro Leu Leu Ala Gln
65                  70                  75                  80

Ala Val Glu Met Leu Ala Ser Ala Asp Arg Leu Arg Gln Phe Glu His
                85                  90                  95

Tyr Glu Lys Phe Leu Leu Lys Lys Gly His Gln Ile Thr Glu Ile Met
            100                 105                 110

Asn Arg Leu Arg Leu Phe Phe Thr Asp Val Leu Lys Val Lys Met Glu
        115                 120                 125

Ala Asp Ala Leu Pro Ala Leu Ala Gln Tyr Leu Met Val Gly Thr Leu
    130                 135                 140

Glu Ala Val Ser Thr Ala Asp Ser Pro Asp Ala Cys Val Pro Val Thr
145                 150                 155                 160

Ser Lys Ile Leu Ala Lys Gln Gln Thr Ile Ala Lys Ser Pro Gly Arg
                165                 170                 175

Leu Asp Glu Glu Glu Tyr Asn Val Ile Arg Ser Arg Phe Leu Thr His
            180                 185                 190

Glu Val Phe Asp Leu Thr Ser Asp Leu Pro Gly Val Gln Pro Phe Met
        195                 200                 205

Asp Met Tyr Tyr Ala Thr Val Pro Arg Ala Asp Ser Thr Gly Trp Cys
    210                 215                 220

Val Tyr Arg Arg Lys Gly Leu Leu Ile His Ala Pro Asp Glu Gln Phe
225                 230                 235                 240

Ser Asp Leu Thr Ile Phe Ser Thr Arg Leu Thr Ala Ser Arg Glu Leu
                245                 250                 255

Gln Leu Val Ala Gly Asp Val Ala Val Ala Cys Phe Asp Leu Met Asp
            260                 265                 270

Val Ser Asp Ile Ala Pro Ser His His Ala Ser Val Gln Glu Glu Arg
        275                 280                 285

Thr Leu Gly Thr Ser Arg Tyr Ser Asn Val Thr Ala Asn Asp His Pro
    290                 295                 300

Leu Val Phe Phe Ser Pro Ser Ala Leu Arg Trp Ala Ile Asp His Ala
305                 310                 315                 320

Cys Thr Asp Ser Leu Val Ser Thr Arg Asn Ile Arg Val Cys Val Gly
                325                 330                 335

Ile Asp Pro Leu Val Thr Arg Trp Thr Arg Asp Gly Val Gln Glu Ala
            340                 345                 350

Ala Ile Leu Met Asp Asp Lys Leu Pro Ser Ala Gly Arg Ala Arg Met
        355                 360                 365

Ala Leu Arg Thr Leu Leu Leu Ala Arg Arg Ser Pro Met Thr Ser Phe
    370                 375                 380

Leu Leu Gly Ala Leu Lys Gln Ser Gly Gly Gln Leu Met Glu His Tyr
385                 390                 395                 400

Arg Cys Asp Ala Ala Asn Arg Tyr Gly Ser Pro Thr Val Pro Val Ser
                405                 410                 415

His Ser Pro Pro Cys Ser Lys Cys Pro Glu Leu Lys Glu Gln Ile Thr
            420                 425                 430

Lys Leu Ser Ser Ser Pro Leu Pro Lys Ile Asp Ser Asn Val Gly Pro
        435                 440                 445

Ala Ala Leu Leu Ser Lys Ile Ala Asp Leu Gln Arg Ala Asn Arg Glu
    450                 455                 460

Leu Ser Leu Lys Leu Val Asp Val Gln Pro Ala Arg Glu Asp His Leu
465                 470                 475                 480

Leu Ala Tyr Leu Asn Glu His Val Cys Val Asn Ala Lys Asp His Glu
                485                 490                 495

Lys Gly Leu Leu Ser Arg Cys Asn Val Ser Gly Asp Ser Ile Ser Ser
            500                 505                 510

Ile Leu Gly Gln Arg Val Lys Asn Arg Glu Arg Phe Glu Thr Arg Leu
        515                 520                 525

Arg His Glu Ala Ser Ala Glu Trp Glu Pro Arg Val Glu Ala Leu Asn
    530                 535                 540

Gln Glu Leu Ala Lys Ala Arg Val Glu Gln Gln Asp Met Met Thr Gln
545                 550                 555                 560

Ser Leu Gln Tyr Leu Asn Glu Arg Asp Glu Leu Leu His Glu Val Asp
                565                 570                 575

Glu Leu Lys Arg Glu Leu Thr Thr Leu Arg Ser Ala Asn Val Arg Leu
            580                 585                 590

Asn Ala Asp Asn His Arg Met Ser Arg Ala Thr Arg Val Gly Asp Ala
        595                 600                 605

Phe Val Ser Asp Val Glu Pro Leu Pro Ser Gly Ile Pro Gly Glu Ser
    610                 615                 620

Lys Pro Ser Met Glu Glu Leu Val Asp Asp Leu
625                 630                 635

<210>  4
<211>  721
<212>  PRT
<213>  Mammalian Orthoreovirus

<400>  4

Met Ala Ser Phe Lys Gly Phe Ser Ala Asn Thr Val Pro Val Ser Lys
1               5                   10                  15

Ala Lys Arg Asp Ile Ser Ser Leu Ala Ala Thr Pro Gly Leu Arg Ser
            20                  25                  30

Gln Ser Phe Thr Pro Ser Val Asp Met Ser Gln Ser Arg Glu Phe Leu
        35                  40                  45

Thr Lys Ala Ile Glu Gln Gly Ser Met Ser Ile Pro Tyr Gln His Val
    50                  55                  60

Asn Val Pro Lys Val Asp Arg Lys Val Val Ser Leu Val Val Arg Pro
65                  70                  75                  80

Phe Ser Ser Gly Ala Phe Ser Ile Ser Gly Val Ile Ser Pro Ala His
                85                  90                  95

Ala Tyr Leu Leu Glu Cys Leu Pro Gln Leu Glu Gln Ala Met Ala Phe
            100                 105                 110

Val Ala Ser Pro Glu Ser Phe Gln Ala Ser Asp Val Ala Lys Arg Phe
        115                 120                 125

Ala Ile Lys Pro Gly Met Ser Leu Gln Asp Ala Ile Thr Ala Phe Ile
    130                 135                 140

Asn Phe Val Ser Ala Met Leu Lys Met Thr Val Thr Arg Gln Asn Phe
145                 150                 155                 160

Asp Val Ile Val Ala Glu Ile Glu Arg Leu Ala Ser Thr Ser Val Ser
                165                 170                 175

Val Arg Thr Lys Glu Ala Lys Val Ala Asp Glu Glu Leu Met Leu Phe
            180                 185                 190

Gly Leu Asp His Arg Gly Pro Gln Gln Leu Asp Val Ser Asp Ala Lys
        195                 200                 205

Gly Ile Met Lys Ala Ala Asp Ile Gln Thr Thr His Asp Val His Leu
    210                 215                 220

Ala Pro Gly Val Gly Asn Ile Asp Pro Glu Ile Tyr Asn Glu Gly Arg
225                 230                 235                 240

Phe Met Phe Met Gln His Lys Pro Leu Ala Ala Asp Gln Ser Tyr Phe
                245                 250                 255

Thr Leu Glu Thr Ala Asp Tyr Phe Lys Ile Tyr Pro Thr Tyr Asp Glu
            260                 265                 270

His Asp Gly Arg Met Ala Asp Gln Lys Gln Ser Gly Leu Ile Leu Cys
        275                 280                 285

Thr Lys Asp Glu Val Leu Ala Glu Gln Thr Ile Phe Lys Leu Asp Ala
    290                 295                 300

Pro Asp Asp Lys Thr Val His Leu Leu Asp Arg Asp Asp Asp His Val
305                 310                 315                 320

Val Ala Arg Phe Thr Lys Val Phe Ile Glu Asp Val Ala Pro Gly His
                325                 330                 335

His Ala Ala Gln Arg Ser Gly Gln Arg Ser Val Leu Asp Asp Leu Tyr
            340                 345                 350

Ala Asn Thr Gln Val Ile Ser Ile Thr Ser Ala Ala Leu Lys Trp Val
        355                 360                 365

Val Lys His Gly Val Ser Asp Gly Ile Val Asn Arg Lys Asn Val Lys
    370                 375                 380

Val Cys Val Gly Phe Asp Pro Leu Tyr Thr Leu Ser Thr His Asn Gly
385                 390                 395                 400

Val Ser Leu Cys Ala Leu Leu Met Asp Glu Lys Leu Ser Val Leu Asn

405                 410                 415

Ser Ala Cys Arg Met Thr Leu Arg Ser Leu Met Lys Thr Gly Arg Asp
            420                 425                 430

Val Asp Ala His Arg Ala Phe Gln Arg Val Leu Ser Gln Gly Tyr Thr
        435                 440                 445

Ser Leu Met Cys Tyr Tyr His Pro Ser Arg Lys Leu Ala Tyr Gly Glu
    450                 455                 460

Val Leu Phe Leu Glu Arg Ser Asn Asp Val Thr Asp Gly Ile Lys Leu
465                 470                 475                 480

Gln Leu Asp Ala Ser Arg Gln Cys His Glu Cys Pro Val Leu Gln Gln
                485                 490                 495

Lys Val Val Glu Leu Glu Lys Gln Ile Ile Met Gln Lys Ser Ile Gln
            500                 505                 510

Ser Asp Pro Thr Pro Val Ala Leu Gln Pro Leu Leu Ser Gln Leu Arg
        515                 520                 525

Glu Leu Ser Ser Glu Val Thr Arg Leu Gln Met Glu Leu Ser Arg Ala
    530                 535                 540

Gln Ser Leu Asn Ala Gln Leu Glu Ala Asp Val Lys Ser Ala Gln Ser
545                 550                 555                 560

Cys Ser Leu Asp Met Tyr Leu Arg His His Thr Cys Ile Asn Gly His
                565                 570                 575

Ala Lys Glu Asp Glu Leu Leu Asp Ala Val Arg Val Ala Pro Asp Val
            580                 585                 590

Arg Arg Glu Ile Met Glu Lys Arg Ser Glu Val Arg Gln Gly Trp Cys
        595                 600                 605

Glu Arg Ile Ser Lys Glu Ala Ala Ala Lys Cys Gln Thr Val Ile Asp
    610                 615                 620

Asp Leu Thr Leu Met Asn Gly Lys Gln Ala Gln Glu Ile Thr Glu Leu
625                 630                 635                 640

Arg Asp Ser Ala Glu Lys Tyr Glu Lys Gln Ile Ala Glu Leu Val Ser
                645                 650                 655

Thr Ile Thr Gln Asn Gln Ile Thr Tyr Gln Gln Glu Leu Gln Ala Leu
            660                 665                 670

Val Ala Lys Asn Val Glu Leu Asp Ala Leu Asn Gln Arg Gln Ala Lys
        675                 680                 685

Ser Leu Arg Ile Thr Pro Ser Leu Leu Ser Ala Thr Pro Ile Asp Ser
    690                 695                 700

Ala Asp Gly Val Ala Asp Leu Ile Asp Phe Ser Val Pro Thr Asp Glu
705                 710                 715                 720

Leu

<210>  5
<211>  62
<212>  PRT
<213>  Artificial Sequence

<220>
<223>  Mammalian Orthoreovirus muNS protein fragment 561-622

<400>  5

Cys Ser Leu Asp Met Tyr Leu Arg His His Thr Cys Ile Asn Gly His
1               5                   10                  15

Ala Lys Glu Asp Glu Leu Leu Asp Ala Val Arg Val Ala Pro Asp Val
            20                  25                  30

Arg Arg Glu Ile Met Glu Lys Arg Ser Glu Val Arg Gln Gly Trp Cys
        35                  40                  45

Glu Arg Ile Ser Lys Glu Ala Ala Ala Lys Cys Gln Thr Val
    50                  55                  60

<210>  6
<211>  5
<212>  PRT
<213>  Artificial Sequence

<220>
<223>  Enterokinase cleavage site

<400>  6

Asp Asp Asp Asp Lys
1               5

<210>  7
<211>  5
<212>  PRT

<213>  Artificial Sequence

<220>
<223>  Factor Xa cleavage site

<400>  7

Ile Glu Asp Gly Arg
1               5


<210>  8
<211>  6
<212>  PRT
<213>  Artificial Sequence

<220>
<223>  Thrombin cleavage site

<400>  8

Leu Val Pro Arg Gly Ser
1               5


<210>  9
<211>  7
<212>  PRT
<213>  Artificial Sequence

<220>
<223>  TEV protease cleavage site

<400>  9

Glu Asn Leu Tyr Phe Gln Gly
1               5


<210>  10
<211>  8
<212>  PRT
<213>  Artificial Sequence

<220>
<223>  PreScission protease cleavage site

<400>  10

Leu Glu Val Leu Phe Gln Gly Pro
1               5


<210>  11
<211>  204
<212>  PRT
<213>  Artificial Sequence

<220>
<223>  Mammalian Orthoreovirus muNS fragment 518-721

<400>  11

Val Ala Leu Gln Pro Leu Leu Ser Gln Leu Arg Glu Leu Ser Ser Glu
1               5                   10                  15

Val Thr Arg Leu Gln Met Glu Leu Ser Arg Ala Gln Ser Leu Asn Ala
            20                  25                  30

Gln Leu Glu Ala Asp Val Lys Ser Ala Gln Ser Cys Ser Leu Asp Met
        35                  40                  45

Tyr Leu Arg His His Thr Cys Ile Asn Gly His Ala Lys Glu Asp Glu
    50                  55                  60

Leu Leu Asp Ala Val Arg Val Ala Pro Asp Val Arg Arg Glu Ile Met
65                  70                  75                  80

Glu Lys Arg Ser Glu Val Arg Gln Gly Trp Cys Glu Arg Ile Ser Lys
                85                  90                  95

Glu Ala Ala Ala Lys Cys Gln Thr Val Ile Asp Asp Leu Thr Leu Met
            100                 105                 110

Asn Gly Lys Gln Ala Gln Glu Ile Thr Glu Leu Arg Asp Ser Ala Glu
        115                 120                 125

Lys Tyr Glu Lys Gln Ile Ala Glu Leu Val Ser Thr Ile Thr Gln Asn
    130                 135                 140

Gln Ile Thr Tyr Gln Gln Glu Leu Gln Ala Leu Val Ala Lys Asn Val
145                 150                 155                 160

Glu Leu Asp Ala Leu Asn Gln Arg Gln Ala Lys Ser Leu Arg Ile Thr
                165                 170                 175

Pro Ser Leu Leu Ser Ala Thr Pro Ile Asp Ser Ala Asp Gly Val Ala
            180                 185                 190

Asp Leu Ile Asp Phe Ser Val Pro Thr Asp Glu Leu
        195                 200

<210>  12
<211>  35
<212>  DNA
<213>  Artificial Sequence

<220>
<223>  Sense primer IGRP

<400>  12
gcgaagcttg gcatggattt ccttcacagg aatgg                                35

<210>  13
<211>  37
<212>  DNA
<213>  Artificial Sequence

<220>
<223>  Antisense primer IGRP

<400>  13
gcgaagcttc tactgactct tctttccgct ttgtttc                                37


<210>  14
<211>  50
<212>  DNA
<213>  Artificial Sequence

<220>
<223>  Sense primer IC

<400>  14
gcataagaat gcggccgcta tcatggcgga agatcacttg ttggcttatc                  50


<210>  15
<211>  35
<212>  DNA
<213>  Artificial Sequence

<220>
<223>  Antisense primer IC

<400>  15
gcgtctagac gcttccacac ggggttccca ctcag                                  35


<210>  16
<211>  29
<212>  DNA
<213>  Artificial Sequence

<220>
<223>  Sense primer muNS-Mi

<400>  16
catgccatgg caccagccgt actgctgtc                                         29


<210>  17
<211>  28
<212>  DNA
<213>  Artificial Sequence

<220>
<223>  Antisense primer muNS-Mi

<400>  17
ttgcggccgc aatcacagat catccacc                                          28


<210>  18
<211>  29

<212>  DNA
<213>  Artificial Sequence

<220>
<223>  Sense primer IC-IGRP

<400>  18
ggagatctcg cggaagatca cttgttggc                                          29


<210>  19
<211>  28
<212>  DNA
<213>  Artificial Sequence

<220>
<223>  Antisense primer IC-IGRP

<400>  19
gggatatcct actgactctt ctttccgc                                           28